# EUROPEAN PATENT APPLICATION

(11) **EP 4 033 201 A1**
(43) Date of publication of application: **27.07.2022**
(21) Application number: 22151715.4
(22) Date of filing: 17.01.2022
(51) Int. Cl.: G01B 9/02056, G01B 9/02091, G01B 9/02

(54) **OCT DEVICE**

(30) Priority: 22.01.2021 JP 2021008395
(71) Applicant: Tomey Corporation, Nagoya-shi, Aichi 451-0051 (JP)
(72) Inventor: KAMO, Takashi, Nagoya-shi, 451-0051 (JP)
(74) Representative: BSB Patentanwälte Schütte & Engelen Partnerschaft mbB

(57) **Abstract**

An OCT device includes a light source that outputs light including a plurality of wavelengths; a division unit that divides light output from the light source into reference light and measurement light; a measurement arm that forms a light path of the measurement light with which a measurement target is irradiated and reflected light from the measurement target, which is generated by the measurement light; a reference arm that forms a light path of the reference light; and a measurement unit that measures the measurement target based on interference light between the reflected light and the reference light. At least one of the measurement arm and the reference arm includes a dispersion unit that disperses light into lights of each wavelength and a dispersed light path portion that forms light paths of dispersed lights, the light paths having a light path length different for each wavelength.

## Description

### CROSS-REFERENCE TO RELATED APPLICATION

This application claims benefit of priority to Japanese Patent Application No. 2021-008395, filed January 22, 2021, the entire content of which is incorporated herein by reference.

### BACKGROUND

### Technical Field:

The present disclosure relates to an OCT device.

### Background Art:

An OCT device (optical coherence tomographic device) is a device that acquires a three-dimensional image based on a tomographic image of a measurement target. Conventionally, in an OCT device, a technique of changing a light path length for each wavelength of light in at least one of a measurement arm and a reference arm is known. For example, JP 2018-47099 A discloses a technique in which a dispersion characteristic of a measurement light path that is a light path of a measurement arm and a dispersion characteristic of a reference light path that is a light path of a reference arm are relatively changed by switching between a state in which a dispersion member is disposed and a state in which a dispersion member is not disposed, in at least one of the measurement light path and the reference light path.

### SUMMARY

In the technique disclosed in JP 2018-47099A, the dispersion characteristic of the light path is adjusted by moving the dispersion member toward the light path or retracting the dispersion member from the light path. Since an interface of the dispersion member is perpendicular to the light path, lights of a plurality of wavelengths travel through the same light path in the configuration disclosed in JP 2018-47099 A. In the dispersion member, a refractive index is different for each wavelength, and thus light path lengths of the lights of a plurality of wavelengths traveling through the same light path are different. In JP 2018-47099 A, such a difference in light path length is used to adjust the dispersion characteristics.

However, in the technique disclosed in JP 2018-47099 A, it is difficult to greatly change a difference in light path for each wavelength of light. Generally, there is a small difference among light path lengths of lights of a plurality of wavelengths traveling on the same light path in a dispersion member. Therefore, in order to greatly change the difference in light path for each wavelength of light using the technique disclosed in JP 2018-47099 A, a very thick dispersion member is required.

Accordingly, the present disclosure provides a technique for easily adjusting a light path length for each wavelength.

The present disclosure provides an OCT device including a light source that outputs light including a plurality of wavelengths; a division unit that divides light output from the light source into reference light and measurement light; a measurement arm that forms a light path of the measurement light with which a measurement target is irradiated and reflected light from the measurement target, which is generated by the measurement light; a reference arm that forms a light path of the reference light; and a measurement unit that measures the measurement target based on interference light between the reflected light and the reference light. A dispersion unit that disperses light into lights of each wavelength and a dispersed light path portion that forms light paths of dispersed lights, the light paths having a light path length different for each wavelength, are provided in at least one of the measurement arm and the reference arm.

That is, in the OCT device, the light including a plurality of wavelengths is dispersed into lights of each wavelength, and then the lights travel through different light paths. Therefore, as compared with the configuration in which a thickness of a dispersion member is adjusted, the light path length of the light of each wavelength is easily adjusted. That is, the light path of the light after being dispersed in the light path for each wavelength of light by the dispersion unit is different for each wavelength. Therefore, this configuration easily greatly change the difference in light path length for each wavelength, as compared with the configuration in which the thickness of the dispersion member is adjusted, by changing a relative positional relationship (distance, posture, or the like) between the dispersion unit and the dispersed light path portion. For this reason, it becomes easy to adjust the light path length for each wavelength, and to design an OCT device having desired characteristics.

### BRIEF DESCRIPTION OF THE DRAWINGS

FIG. 1 is a diagram illustrating a configuration of an OCT device according to an embodiment of the present disclosure;
FIGS. 2A to 2C are diagrams for explaining an increase in measurement range;
FIG. 3 is a diagram illustrating a configuration example of a light path length conversion unit;
FIG. 4 is a diagram illustrating a configuration example of the light path length conversion unit;
FIG. 5 is a diagram illustrating a configuration example of the light path length conversion unit;
FIG. 6 is a diagram illustrating a blazed diffraction grating;
FIG. 7 is a diagram illustrating a configuration example of the light path length conversion unit;
FIG. 8 is a diagram illustrating a configuration example of the light path length conversion unit;
FIG. 9 is a diagram illustrating a configuration example of the light path length conversion unit;
FIG. 10 is a diagram illustrating a configuration example of the light path length conversion unit;
FIG. 11 is a diagram illustrating a configuration of an OCT device according to an embodiment of the present disclosure; and
FIG. 12 is a diagram illustrating a configuration example of the light path length conversion unit.

### DETAILED DESCRIPTION

An embodiment of the present disclosure will be described in the following order.
(1) Configuration of OCT device:
(2) Configuration of light path length conversion unit:
(3) Other embodiments:

### (1) Configuration of OCT device

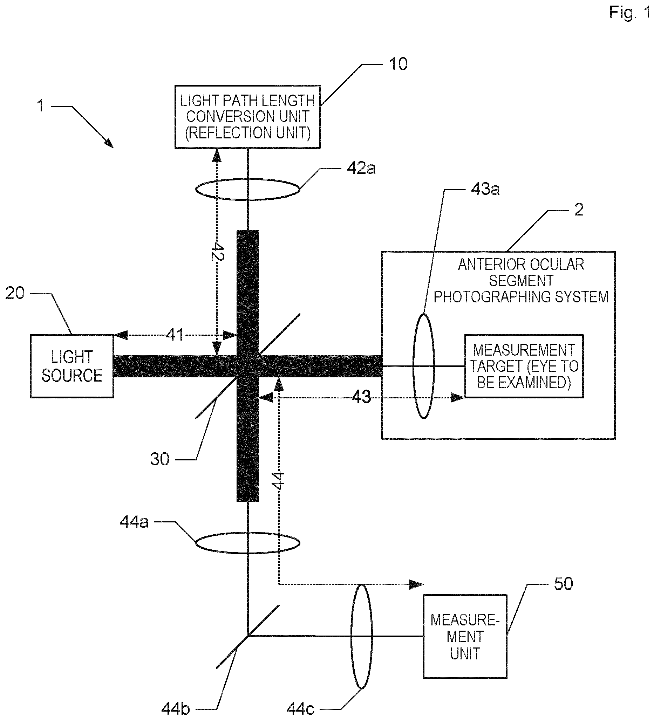

Hereinafter, an ophthalmic device including an OCT device 1 according to an embodiment of the present disclosure will be described. FIG. 1 is a diagram schematically illustrating a configuration of an ophthalmic device. The ophthalmic device includes an OCT device 1 and an anterior ocular segment photographing system 2. The anterior ocular segment photographing system 2 includes an alignment optical system, a light projecting optical system, a photographing optical system, and the like (not illustrated). The alignment optical system includes a configuration for moving and positioning the anterior ocular segment photographing system 2 and the OCT device 1 with respect to a subject's eye to be examined.

The light projecting optical system is an optical system for projecting light to the eye to be examined, and the type, wavelength, light projecting direction, and the like of a light source are not limited. The photographing optical system is an optical system that guides light from the eye to be examined to an imaging element to photograph the eye to be examined. A photographing target is not limited, and may be various parts of the eye to be examined. Note that the anterior ocular segment photographing system 2 only needs to be able to photograph the eye to be examined, and can be realized by various known configurations as described in JP 2018-47099 A and the like. In addition, the respective optical systems may share at least some of optical components. In the ophthalmic device according to the present embodiment, the eye to be examined is photographed by the anterior ocular segment photographing system 2, and various examinations can be performed.

On the other hand, the ophthalmic device can take a tomographic image of the fundus of the eye to be examined as a measurement target by the OCT device 1. The OCT device 1 is a device that decomposes light output from a light source into reference light and measurement light, irradiates the eye to be examined as the measurement target with the measurement light, and generates a tomographic image of the measurement target by interference light between reflected light obtained by reflection by the eye to be examined and the reference light.

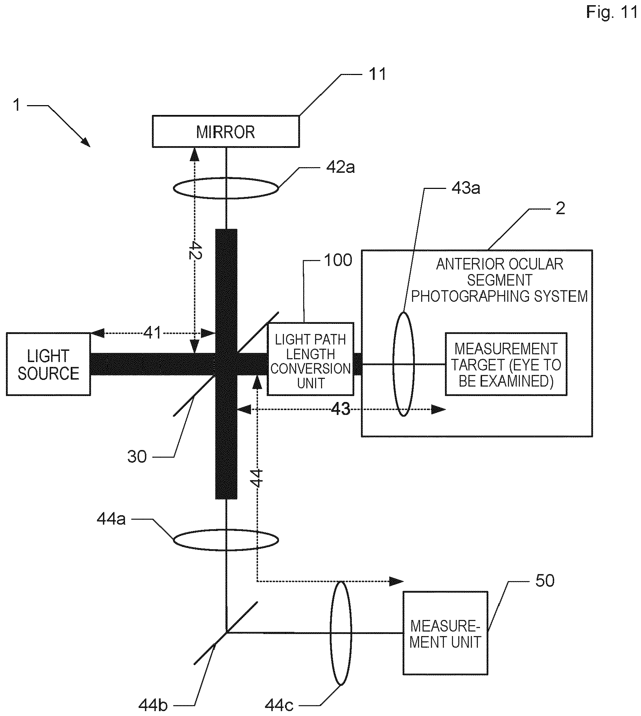

The OCT device 1 generates interference light by a Michelson's interferometer. For this purpose, the OCT device 1 includes a light path length conversion unit 10, a light source 20, a division unit 30, and an optical member that forms a plurality of light paths 41 to 44. In FIG. 1 and FIG. 11 which will be described later, a black rectangle constituting a part of the light paths 41 to 44 schematically illustrates an optical fiber constituting a part of the light paths 41 to 44. In the present embodiment, the light source outputs light in a predetermined wavelength range, and has a center wavelength of 840 nm and a full width at half maximum of 60 nm (that is, 840 ± 30 nm). The division unit 30 is an optical member that divides the light output from the light source 20 into the reference light and the measurement light, and can be composed of, for example, a filter coupler or the like. That is, the division unit 30 divides the light output from the light source 20 and traveling through the light path 41 into two systems, i.e., the reference light and the measurement light, and causes the reference light to travel to the light path 42 and the measurement light to travel to the light path 43.

Furthermore, the reference light traveling through the light path 42 and reflected by the light path length conversion unit 10 travels through the light path 42 again, and reaches the division unit 30. The reflected light traveling through the light path 43 and reflected by the measurement target travels through the light path 43 again, and reaches the division unit 30. The division unit 30 combines the reference light and reflected light reaching the division unit 30, and causes the combined light to travel to the light path 44.

In the present embodiment, the reference light obtained by the division by the division unit 30 travels through the light path 42, the light path length conversion unit 10, the light path 42, the division unit 30, and the light path 44 in this order, and reaches the measurement unit 50. Therefore, in the present embodiment, the light path 42, the light path length conversion unit 10, and the light path 44 form a light path of the reference light. In the present embodiment, the light path of the reference light is referred to as a reference arm. In addition, the measurement light obtained by the division by the division unit travels through the light path 43, the measurement target, the light path 43, the division unit 30, and the light path 44 in this order, and reaches the measurement unit 50. Therefore, in the present embodiment, the light path 43 and the light path 44 form light paths of the measurement light and the reflected light. In the present embodiment, a light path of the measurement light and the reflected light is referred to as a measurement arm.

The light path 41 is a part that causes the light from the light source 20 to travel to the division unit 30, and may be formed of various optical components. Since the light is divided into the measurement light and the reference light by the division unit 30, the light path 41 is a light path of the measurement light and the reference light.

The light path 42 is a light path through which the reference light obtained by the division unit 30 travels toward the light path length conversion unit 10, and is a light path through which the reference light traveling in an opposite direction by being reflected by a reflection unit of the light path length conversion unit 10 travels toward the division unit 30. The light path 42 may be formed of various optical components. In FIG. 1, a collimator 42a among the optical components is illustrated. The light path length conversion unit 10 will be described in detail later.

The light path 43 is a light path through which the measurement light obtained by the division unit 30 travels toward the measurement target, and is a light path through which the reflected light reflected by the measurement target and traveling in an opposite direction to that of the measurement light travels toward the division unit 30. The light path 43 may be formed of various optical components. In FIG. 1, a collimator 43a among the optical components is illustrated. Note that, in the present specification, the light returning to the division unit 30 on the light path 42 is simply referred to as reference light, but the measurement light returning to the division unit 30 on the light path 43 is referred to as reflected light.

The light path 44 is a light path through which the reflected light and the reference light combined by the division unit 30 travel toward the measurement unit 50. The light path 44 may be formed of various optical components, and, in FIG. 1, a collimator 44a, a diffraction grating 44b, and a lens 44c are illustrated. That is, the light traveling through the light path 44 is collimated by the collimator 44a, and is dispersed into lights of each wavelength by the diffraction grating 44b. The dispersed lights are incident on the measurement unit 50 via the lens 44c.

The measurement unit 50 includes a plurality of sensors that detect intensity of the incident lights and are disposed at different positions. Since the lights incident on the measurement unit 50 are dispersed by the diffraction grating 44b, and travel through the light paths for each wavelength as a result of the dispersion, the positions of the sensors that detect the lights in the measurement unit 50 correspond to the wavelengths of the lights. Therefore, in the measurement unit 50, the intensity of the light of each wavelength can be detected. Since the lights incident on the measurement unit 50 are in a state in which the reflected light and the reference light are combined, the measurement unit 50 detects interference light between the reflected light and the reference light.

The measurement unit 50 includes a calculation unit (not illustrated), and the calculation unit can generate a tomographic image of the measurement target by performing a known calculation on the basis of the detected interference light. That is, the measurement target can be measured. As described above, the present embodiment is SD-OCT (Spectral Domain-Optical Coherence Tomography) in order to generate interference light and measure the interference light dispersed by the diffraction grating 44b using a Michelson's interferometer. Of course, the configuration illustrated in FIG. 1 is an example, and the optical components included in the OCT device 1 may be different, and the OCT device 1 may be composed of OCT of another Fourier domain, for example, SS-OCT.

As described above, the measurement unit 50 performs measurement by a known calculation on the basis of the interference light of each wavelength, and the calculation may include correction of wavelength dispersion. In the present embodiment, the correction is used to increase a measurement range. Of course, whether to perform the wavelength dispersion correction may be selectable by a user.

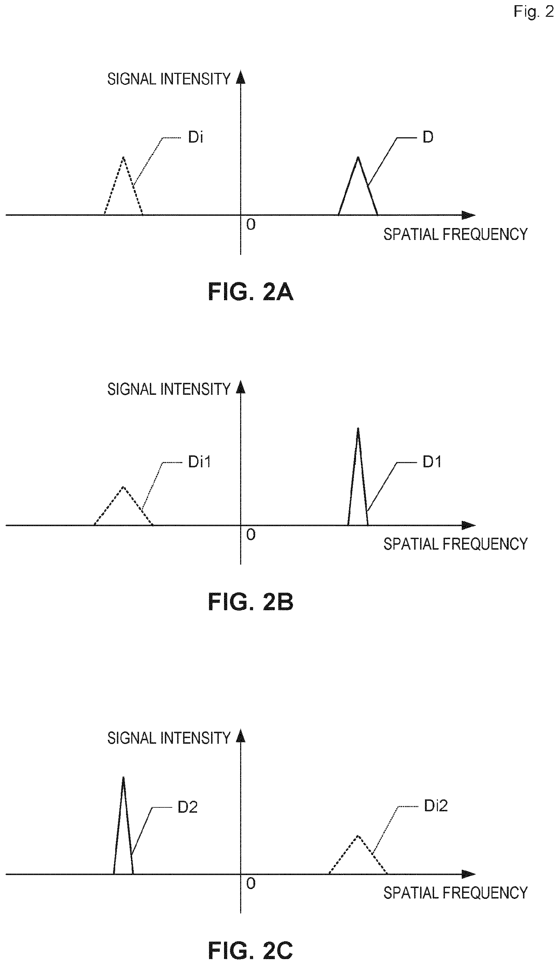

FIGS. 2A to 2C are diagrams schematically illustrating correction of wavelength dispersion and an increase in measurement range. FIG. 2A schematically illustrates intensity of a signal (hereinafter referred to as interference signal) obtained by Fourier-transforming intensity of the interference light for each position of the sensor detected by the measurement unit 50 (the position of the sensor corresponds to the wavelength of the light). In FIGS. 2A to 2C, the horizontal axis represents spatial frequency, and the vertical axis represents the intensity of the interference signal. Since the intensity of the interference light is detected in the measurement unit 50, the intensity of the interference light changes according to light path lengths of the light traveling through the measurement arm and the light traveling through the reference arm.

When the light path length of the reference light is equal to the light path lengths of the measurement light and the reflected light, the light traveling through the measurement arm and the light traveling through the reference arm interfere and intensify each other in the lights of all the wavelengths. Therefore, the intensity of the interference signal after the Fourier transform becomes a distribution having a peak at a spatial frequency of 0.

On the other hand, in a case where the light path length of the reference light is different from the light path lengths of the measurement light and the reflected light, if a difference in light path length is an integral multiple of the wavelength, the light traveling through the measurement arm and the light traveling through the reference arm interfere and intensify each other. If the difference in light path length is an integral multiple of the wavelength + a half wavelength, the light traveling through the measurement arm and the light traveling through the reference arm interfere and cancel each other. Therefore, in a case where there is a difference in light path length, the intensity of the interference light differs for each wavelength, and the intensity of the interference light differs for each wavelength according to the difference in light path length.

In the example illustrated in FIG. 2A, the intensity of the interference signal in a case where the light path length of the light traveling through the measurement arm is longer than the light path length of the light traveling through the reference arm is illustrated as a distribution D. In a case where the light path length of the light traveling through the measurement arm is longer than the light path length of the light traveling through the reference arm, the distribution D appears in a region where the spatial frequency is greater than 0. In a case where there is a difference in light path length of light of each wavelength in the reference arm or the measurement arm, the spatial frequency of the intensity of the interference light is different for each wavelength of light, and thus, the distribution of the interference signal is a broad distribution spreading in a spatial frequency direction.

In a Fourier-domain OCT, the interference signal with respect to the spatial frequency is analyzed using the Fourier transform as described above, but, at this time, a complex conjugate signal of the interference signal occurs with the Fourier transform. In FIG. 2A, a distribution Di of the complex conjugate signal is indicated by a broken line. Such a complex conjugate signal is generated at a position symmetric with the distribution D about a 0 point on the axis of the spatial frequency. Therefore, on the basis of a state in which the difference between the light path length of the light traveling through the reference arm and the light path length of the light traveling through the measurement arm is equal, a case in which the difference in light path length becomes longer by a distance L and a case in which the difference in light path length becomes shorter by the distance L cannot be distinguished from a profile of the interference signal illustrated in FIG. 2A. Therefore, in an example illustrated in FIG. 2A, measurement needs to be performed only when the light path length of the light traveling through the reference arm is equal to or longer than the light path length of the light traveling through the measurement arm, or when the light path length of the light traveling through the reference arm is equal to or shorter than the light path length of the light traveling through the measurement arm. That is, the measurement range is limited.

Therefore, the measurement unit 50 according to the present embodiment distinguishes between the interference signal and the complex conjugate signal by a dispersion encoded full range (DEFR) algorithm. In addition, the measurement unit 50 increases the measurement range by distinguishing between the interference signal and the complex conjugate signal. The DEFR algorithm is known as, for example, Optics Express Vol. 17, Issue 1, pp. 7-24 (2009). In the DEFR algorithm, when the wavelength dispersion is corrected for the interference signal after the Fourier transform, the distribution of the interference signal becomes narrower and the distribution of the complex conjugate signal becomes wider.

Therefore, for example, when the distribution D of the interference signal exists on a positive side of the spatial frequency and the distribution Di of the complex conjugate signal exists on a negative side as illustrated in FIG. 2A, the positive-side interference signal has a narrow distribution Di and the negative-side complex conjugate signal has a wide distribution Dii as illustrated in FIG. 2B. On the other hand, if, in the distributions illustrated in FIG. 2A, the distribution existing on the negative side of the spatial frequency is for the interference signal, and the distribution existing on the positive side is for the complex conjugate signal, the negative-side interference signal has a narrow distribution D₂, and the positive-side complex conjugate signal has a wide distribution Di₂, as illustrated in FIG. 2C.

As described above, when the wavelength dispersion is corrected, the interference signal corresponding to a true signal from the measurement target and the complex conjugate signal inevitably generated according to the Fourier transform can be distinguished from each other. Therefore, after correcting the wavelength dispersion, the measurement unit 50 removes the complex conjugate signal by leaving the narrowed distribution and removing the widened distribution. By leaving the interference signal in this way, it is possible to specify a spatial frequency region where the interference signal exists. Therefore, the measurement unit 50 can generate a tomographic image of the measurement target after specifying whether the measurement target exists at a position where the light path length becomes longer than that at the position where the reference light is reflected or at a position where the light path length becomes shorter than that at the position where the reference light is reflected, depending on whether the interference signal exists on either the positive side or the negative side. When a tomographic image of the measurement target is generated by the measurement unit 50, the measurement unit 50 stores data indicating the tomographic image in a storage medium (not illustrated). The stored data indicating the tomographic image is used for displaying the tomographic image, various examinations, and the like.

As illustrated in FIG. 2A, the above DEFR algorithm uses the fact that the distribution D becomes broad due to the spatial frequency of the intensity of the interference light being different for each wavelength of light in a case where there is a difference in light path length among the lights of each wavelength in the reference arm or the measurement arm. Therefore, if the distribution is not sufficiently broad, the DEFR is not established. In the present embodiment, it is assumed that, when the wavelength of the light output from the light source 20 is in a narrow wavelength range centered on a center wavelength of 840 nm, the distribution of the interference signal is not sufficiently broad.

Furthermore, the wavelength range of light used in the OCT device 1 may be slightly different for each individual depending on individual differences of the light source 20, setting differences with respect to the OCT device 1, and the like. Since the interference signal has a distribution reflecting a change in intensity of interference according to a difference in wavelength, when the wavelength range of light used in the OCT device 1 is different, the interference signal obtained by the same measurement target may be different depending on the individual of the OCT device 1. For this reason, tomographic images obtained by processing by the DEFR algorithm may also be different.

Therefore, in the present embodiment, the light path length conversion unit 10 is provided in order to generate an interference signal having a sufficiently broad distribution easily, and in order to adjust finely the light path length for each wavelength of light according to a slight difference in wavelength range of light. Since the OCT device 1 according to the present embodiment can finely adjust the light path length for each wavelength of light by the light path length conversion unit 10, it is not necessary to design the OCT device 1 so as not to cause individual differences of the light source 20, setting differences, and the like. Therefore, the OCT device 1 can be easily designed.

### (2) Configuration of light path length conversion unit

In the present embodiment, the light path length conversion unit 10 is provided in the reference arm. Specifically, the light path length conversion unit 10 includes a dispersion unit that disperses light in a traveling direction different for each wavelength, and a dispersed light path portion that forms a light path having a light path length different for each wavelength. Further, in the present embodiment, the light path length conversion unit 10 includes a reflection unit that reflects the reference light.

The reference light incident on the light path length conversion unit 10 is dispersed into lights of each wavelength, and travels through different light paths in a dispersed state, so that the reference light travels through light paths having different light path lengths for each wavelength. Then, the reference light is reflected inside the light path length conversion unit 10, and is output to the light path 42 again.

The configuration for dispersing light for each wavelength may be various, but, in the present embodiment, the light is dispersed into lights of each wavelength by the optical member. Here, the optical member is referred to as a first optical member.

The dispersed light path portion that forms light paths having different light path lengths for each wavelength may also have various configurations. In the present embodiment, the dispersed light path portion includes a traveling direction conversion unit that receives lights of each wavelength dispersed by the first optical member, converts traveling directions of the lights of each wavelength into opposite directions while maintaining a dispersed state, and returns the lights to the first optical member. The traveling direction conversion unit can be composed of a plurality of optical members. In the present embodiment, the traveling direction conversion unit is composed of a second optical member that is a member of the same type as the first optical member, and a reflection member that reflects light.

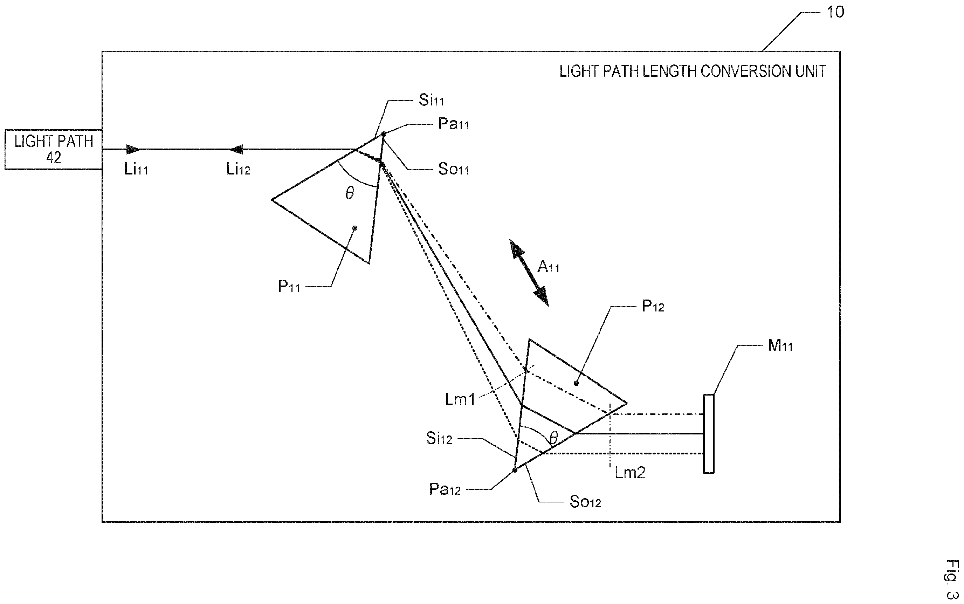

FIG. 3 is a diagram schematically illustrating a part of a configuration according to an example of the light path length conversion unit 10. In the configuration illustrated in FIG. 3, the first optical member is composed of a prism P₁₁. The second optical member is composed of a prism P₁₂, and the reflection member is composed of a mirror M11. The light path length conversion unit 10 includes various members in addition to these members. For example, in the present embodiment, the light path length conversion unit 10 includes a moving mechanism (not illustrated) for moving the prism P12 and the mirror M11 forward and backward toward the prism P11.

In the configuration illustrated in FIG. 3, light Li₁₁ incident on the light path length conversion unit 10 from the light path 42 travels toward the prism P₁₁. The light reaching the prism P₁₁ is refracted at an interface of the prism P₁₁. At this time, the light is dispersed, and the dispersed lights travel in different traveling directions for each wavelength. Further, when the lights of each wavelength reach an interface on an opposite side of the prism P₁₁, the lights are refracted again, and travel in the air. In FIG. 3, routes of the lights having different wavelengths are indicated by dot-dash lines, solid lines, and broken lines. The lights traveling through the light paths indicated by the dot-dash lines have a longer wavelength than those of the lights traveling through the light paths indicated by the solid lines, and the lights traveling through the light paths indicated by the solid lines have a longer wavelength than those of the lights traveling through the light paths indicated by the broken lines. Of course, a larger number of the lights of each wavelength are continuously distributed, but only three light paths are extracted and schematically illustrated in FIG. 3.

When the lights output from the prism P₁₁ reach the prism P₁₂, the lights are refracted at an interface, travel inside the prism P₁₂, and are refracted again at an interface on an opposite side, and the lights of each wavelength travel toward the mirror M₁₁ in a state of being parallel to each other. When the lights of each wavelength reach the mirror M₁₁, the lights are reflected by the mirror M₁₁, become opposite, in traveling direction, to the lights before reflection, and return to the same light paths as the former light paths. That is, the lights reflected by the mirror M₁₁ pass through the prism P₁₂ and the prism P₁₁, and, at a stage of being output from the prism P₁₁, the lights traveling on the light paths different for each wavelength (dispersed lights) return to the state before dispersion. Then, light Li₁₂ output from the prism P₁₁ returns to the light path 42.

Note that the above configuration is an example, and, for example, in a case where a Mach-Zehnder interferometer is used, the reference light traveling through the reference arm may not return to the same light path. Note that the light incident on the light path length conversion unit 10 from the light path 42 and the light incident on the light path 42 from the light path length conversion unit 10 are expressed by one line, but may be parallel lights having a width (hereinafter, the same applies to other embodiments).

In the present embodiment, the prisms P₁₁ and P₁₂ are the same medium and have the same shape. In the example illustrated in FIG. 3, the prisms P₁₁ and P₁₂ are triangular prisms, and axes of the triangular prisms are parallel to each other. In addition, a plane perpendicular to the axes of the triangular prisms (a plane parallel to the paper surface of FIG. 3) is parallel to an optical axis of the light Li₁₁. Further, an incident surface Si₁₁ of the light to the prism P₁₁ and an outgoing surface So₁₂ of the light from the prism P₁₂ are oriented in parallel, and an outgoing surface So₁₁ of the light from the prism P₁₁ and an incident surface Si₁₂ of the light on the prism P₁₂ are oriented in parallel. The mirror M₁₁ is directed in a direction perpendicular to the light output from the prism P₁₂.

Therefore, the light incident on the prism P₁₁ and the light directed from the prism P₁₂ toward mirror M₁₁ are parallel to each other. When the light passes through the prisms P₁₁ and P₁₂, the light is directed toward the mirror M₁₁ while its dispersed state is maintained for each wavelength. When the light of each wavelength directed to the mirror M₁₁ reaches the mirror M₁₁, the light is reflected and travels in an opposite direction. As described above, in the present embodiment, the light dispersed by the prism P₁₁, which is the first optical member, is converted to be directed in the opposite direction while its dispersed state is maintained by the prism P₁₂, which is the second optical member, and the mirror M₁₁, which is the reflection member, and is returned to the prism P₁₁, which is the first optical member. Since the light output from the prism P₁₁ and the light returning to the prism P₁₁ are in opposite directions, when the returned light passes through the prism P₁₁, the light returns to a state of not being dispersed for each wavelength.

As described above, in the light path length conversion unit 10, the light input from the light path 42 is dispersed into lights of each wavelength. In addition, the dispersed lights of each wavelength travel through different light paths. In the present embodiment, the dispersion unit and the dispersed light path portion having such functions are realized by the prisms P₁₁ and P₁₂ and the mirror M₁₁, and the light path length conversion unit 10 can be configured with a simple configuration.

In the present embodiment, the dispersed lights travel through different light paths, and thus the light path lengths of the dispersed lights are different for each wavelength. In the configuration illustrated in FIG. 3, the light path lengths between two-dot chain lines Lm1 and Lm2 are different for each wavelength. Furthermore, in the present embodiment, the light path length for each wavelength can be changed. The configuration for changing the light path length may be realized by various configurations, and, for example, it is possible to change the light path length for each wavelength of dispersed light by changing at least one of a distance between or directions of the dispersion unit and the traveling direction conversion unit.

In the present embodiment, the prism P₁₂ and the mirror M₁₁ constituting the traveling direction conversion unit can be integrally moved. Specifically, the prism P₁₂ and the mirror M₁₁ are connected to the moving mechanism (not illustrated). The moving mechanism can move the prism P₁₂ and the mirror M₁₁ while maintaining a state in which a relative positional relationship between the prism P₁₂ and the mirror M₁₁ does not change and the outgoing surface So₁₁ of the prism P₁₁ and the incident surface Si₁₂ of the prism P₁₂ are parallel to each other. An arrow A₁₁ illustrated in FIG. 3 indicates a movable direction of the prism P₁₂ and the mirror M₁₁.

When the prism P₁₂ and the mirror M₁₁ are moved in the movable direction, a distance between the prisms P₁₁ and P₁₂ varies. When the distance between the prisms P₁₁ and P₁₂ varies, a position where lights of the same wavelength are incident on the incident surface Si₁₂ varies. For example, when the prism P₁₂ and the mirror M₁₁ move in a direction of approaching the prism P₁₁ along the arrow A₁₁ from the state illustrated in FIG. 3, the lights indicated by the dot-dash line, the solid line, and the broken line are incident on the incident surface Si₁₂ at a position farther from a vertex Pa₁₂ of the prism P₁₂ than the state illustrated in FIG. 3. As a result, for example, the light path length of the light of each wavelength becomes long in the prism P₁₂. As described above, when the prism P₁₂ and the mirror M₁₁ are moved in the direction of the arrow A₁₁, the light path length changes. At this time, a degree of change in light path length varies for each wavelength. Therefore, according to the present embodiment, the light path length can be changed in a different change amount for each wavelength.

The moving mechanism can be realized by various mechanisms for reciprocating an object in a linear direction. For example, a linear reciprocating motion may be realized by a ball screw mechanism or the like, or a linear reciprocating motion may be realized by a power transmission mechanism such as a slider crank mechanism or a cam. Note that the configuration for changing the light path length is not limited to the configuration that reciprocates the prism P₁₂ and the mirror M₁₁ in the linear direction. For example, vertexes Pa₁₁ and Pa₁₂ of the prisms P₁₁ and P₁₂ may be set as rotation axes, and orientations of the prisms P₁₁ and P₁₂ may be changed so as to rotate by the same angle in the same rotation direction. That is, the prisms P₁₁ and P₁₂ may be rotated while maintaining a state in which the outgoing surface So₁₁ of the prism P₁₁ and the incident surface Si₁₂ of the prism P₁₂ are parallel to each other.

Further, the prism P₁₁ may be reciprocatable in the linear direction. Needless to say, a mechanism that enables the prisms P₁₁ and P₁₂ to rotate or move in the linear direction may be used in combination. Note that the moving mechanism may be configured to be manually operated by the user, or may be configured to be operated in accordance with a control instruction from the measurement unit 50 or the like. In the latter case, for example, power of a motor or the like can be transmitted to the moving mechanism, and the motor or the like is operated in accordance with the control instruction.

Furthermore, shapes of the prisms P₁₁ and P₁₂ and the mirror M₁₁ are not limited to those in the configuration illustrated in FIG. 3. For example, the shapes of the prisms P₁₁ and P₁₂ may not be completely the same. That is, in the prisms P₁₁ and P₁₂, angles θ at the vertexes Pa₁₁ and Pa₁₂ only need to be the same, and the outgoing surface So₁₁ of the prism P₁₁ and the incident surface Si₁₂ of the prism P₁₂ only need to be parallel to each other. Therefore, for example, triangles formed by the prisms P₁₁ and P₁₂ in FIG. 3 may not be congruent. Further, when the mirror M₁₁ is sufficiently larger than that illustrated in FIG. 3, the prism P₁₂ may be moved instead of integrally moving the prism P₁₂ and the mirror M₁₁.

As described above, various modifications can be adopted in the present embodiment, but, in any case, according to the present embodiment, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

### (3) Other embodiments

The above embodiment is an example for carrying out the present disclosure, and various other embodiments can also be adopted. Therefore, at least a part of the configuration of the above-described embodiment may be omitted, or replaced.

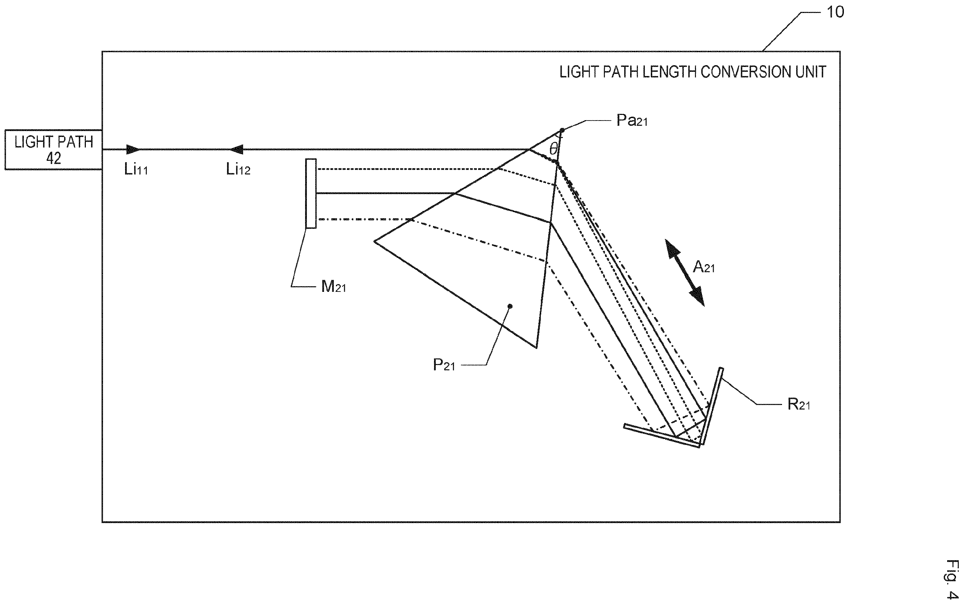

For example, when the dispersed light path portion is composed of the traveling direction conversion unit, the dispersed light path portion and the dispersion unit may share a member. FIG. 4 is a diagram schematically illustrating an embodiment in which the first optical member is composed of a prism P₂₁, and the traveling direction conversion unit is composed of the prism P₂₁, a retroreflector R₂₁, and a mirror M₂₁.

FIG. 4 is a diagram schematically illustrating a part of a configuration extracted from the light path length conversion unit 10 according to the present embodiment. The light path length conversion unit 10 includes various members in addition to members illustrated in FIG. 4. Elements similar to those in FIG. 3 are denoted by the same reference numerals as those in FIG. 3. Further, the light path length conversion unit 10 illustrated in FIG. 4 includes a moving mechanism (not illustrated) for moving the retroreflector R21 forward and backward in a direction indicated by an arrow A21 toward the prism P21. Note that the retroreflector R21 is a device that reflects light incident from an arbitrary direction and causes the light to travel in a direction parallel to and opposite to an incident direction. Therefore, the retroreflector R₂₁ is a conversion optical member that converts the lights of each wavelength output from the prism P₂₁ as the first optical member into lights traveling in an opposite traveling direction and on different light paths, and returns the lights to the prism P₂₁ as the first optical member.

In the configuration illustrated in FIG. 4, light Li11 incident on the light path length conversion unit 10 from the light path 42 travels toward the prism P21. The light reaching the prism P21 is refracted at an interface of the prism P21. At this time, the light is dispersed, and the dispersed lights travel in different traveling directions for each wavelength. Further, when the lights of each wavelength reach an interface on an opposite side of the prism P21, the lights are refracted again, and travel in the air. Also in FIG. 4, routes of the lights having different wavelengths are indicated by dot-dash lines, solid lines, and broken lines. The lights traveling through the light paths indicated by the dot-dash lines have a longer wavelength than those of the lights traveling through the light paths indicated by the solid lines, and the lights traveling through the light paths indicated by the solid lines have a longer wavelength than those of the lights traveling through the light paths indicated by the broken lines. Of course, a larger number of the lights of each wavelength are continuously distributed, but only three light paths are extracted and schematically illustrated also in FIG. 4.

When the lights output from the prism P₂₁ reach the retroreflector R₂₁, the lights are reflected by the retroreflector R₂₁ and travel in a direction parallel to the incident direction and opposite to the incident direction. The lights reach the prism P₂₁, and the lights reaching the prism P₂₁ are refracted at the interface of the prism P₂₁. When the lights traveling in the prism P₂₁ reach an interface on an opposite side of the prism P₂₁, the lights are refracted again. The lights of each wavelength travel toward the mirror M₂₁ as the reflection member in a state of being parallel to each other. When the lights of each wavelength reach the mirror M₂₁, the lights are reflected by the mirror M₂₁, become opposite, in traveling direction, to the lights before reflection, and return to the same light paths as the former light paths. That is, the lights reflected by the mirror M₂₁ pass through the prism P₁₂, are reflected by the retroreflector R₂₁, and are incident on the prism P₂₁ again. When the lights incident on the prism P₂₁ are output from the prism P₂₁, the lights (dispersed lights) traveling through light paths different for each wavelength return to the state before dispersion. Then, the light Li₁₂ output from the prism P₂₁ returns to the light path 42.

Also in an example illustrated in FIG. 4, the prism P₂₁ is a triangular prism. In addition, a plane perpendicular to the axes of the triangular prisms (a plane parallel to the paper surface of FIG. 4) is parallel to an optical axis of the light Li₁₁. The mirror M₂₁ is oriented in a direction perpendicular to the light output from the prism P₂₁ and traveling toward the mirror M₂₁.

As described above, in the configuration of the present embodiment, the prism P₁₂ and the mirror M₁₁ are excluded from the configuration illustrated in FIG. 3, and the retroreflector R₂₁ and the mirror M₂₁ are disposed instead. Since the retroreflector R₂₁ is a device that causes light to travel in the opposite direction, the configuration illustrated in FIG. 4 and the configuration illustrated in FIG. 3 are substantially almost equivalent to each other, although there is a difference that the light paths of the embodiment illustrated in FIG. 4 are folded back between the prisms P₁₁ and P₁₂ illustrated in FIG. 3.

As described above, also in the configuration illustrated in FIG. 4, the light dispersed by the prism P₂₁, which is the first optical member, is reciprocated by the retroreflector R₂₁, the prism P₂₁, and the mirror M₂₁, so that the light is converted to be directed in the opposite direction while its dispersed state is maintained, and is returned to the prism P₂₁, which is the first optical member. Then, the lights traveling from the prism P₂₁ toward the light path 42 again return to a state of not being dispersed for each wavelength.

Also in the configuration illustrated in FIG. 4, the dispersed lights travel through the light paths for each wavelength. Also in the present embodiment, the dispersion unit and the dispersed light path portion having such functions are realized by the prism P₂₁, the retroreflector R₂₁, and the mirror M₂₁, and the light path length conversion unit 10 can be configured with a simple configuration.

Furthermore, also in the configuration illustrated in FIG. 4, the dispersed lights travel through different light paths, and thus the light path lengths of the dispersed lights are different for each wavelength, and the light path lengths can be changed. Specifically, the retroreflector R₂₁ is connected to a moving mechanism (not illustrated). The moving mechanism can linearly reciprocate the retroreflector R₂₁ along the direction of the arrow A₂₁, and can vary a distance between the retroreflector R₂₁ and the prism P₂₁. When the distance between the retroreflector R₂₁ and the prism P₂₁ varies, the light path length of the light of each wavelength changes. At this time, a degree of change in light path length varies for each wavelength. Therefore, according to the present embodiment, the light path length can be changed in a different change amount for each wavelength. Note that, also in the configuration illustrated in FIG. 4, the moving mechanism can be realized by various mechanisms for reciprocating an object in a linear direction, and the prism P₂₁ may be rotated with a vertex Pa₂₁ of the prism P₂₁ as a rotation axis.

Also in the present embodiment, with the above configuration, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

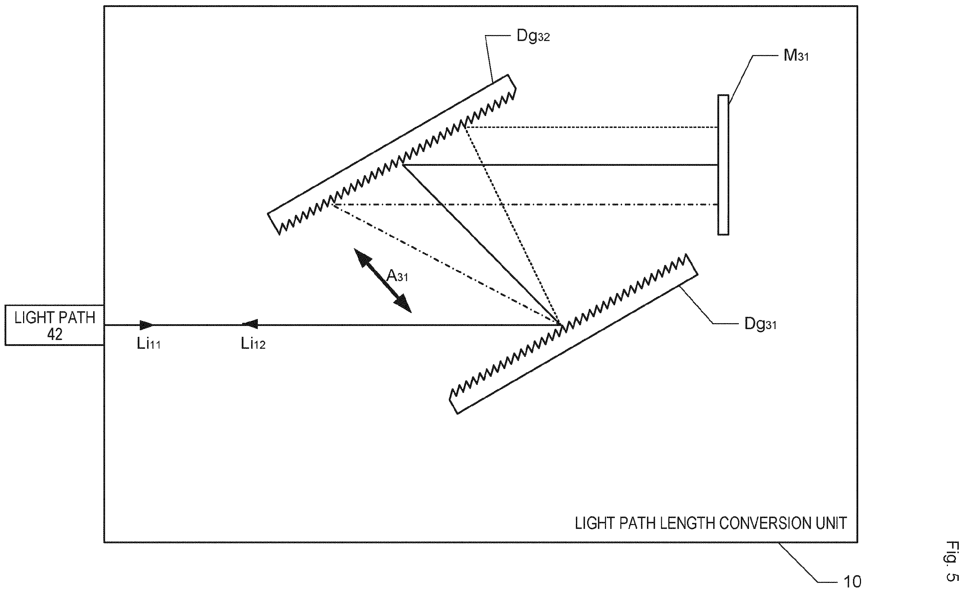

In the above-described embodiment, the dispersion unit is composed of a prism, but may be composed of another optical component. FIG. 5 is a diagram illustrating an example in which the first optical member as the dispersion unit is composed of a diffraction grating. Also in a configuration illustrated in FIG. 5, the light path length conversion unit 10 includes a dispersion unit and a dispersed light path portion, the dispersion unit is composed of a first optical member, and the dispersed light path portion is composed of a traveling direction conversion unit including a second optical member that is a member of the same type as the first optical member and a reflection member that reflects light.

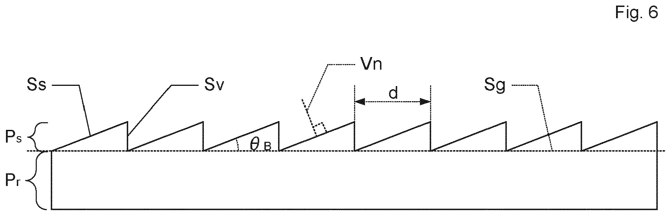

In the configuration illustrated in FIG. 5, the dispersion unit is a first blazed diffraction grating Dg₃₁ as the first optical member, and the dispersed light path portion includes a second blazed diffraction grating Dg₃₂ as the second optical member and a mirror M₃₁ as the reflection member. As illustrated in FIG. 6, the blazed diffraction grating is an optical member in which a step surface is formed on one surface of a rectangular parallelepiped member. In a configuration example illustrated in FIG. 6, a plurality of wedge-shaped portions Ps are formed on one surface Sg of a rectangular parallelepiped portion Pr. The wedge-shaped portion Ps has a step surface Ss inclined by a blaze angle θB with respect to the surface Sg and a vertical surface Sv perpendicular to the surface Sg. The wedge-shaped portion Ps is repeatedly formed on the surface Sg at groove spacing d. In the present embodiment, the surface Sg obtained by excluding the wedge-shaped portion Ps in the blazed diffraction grating is referred to as a diffraction grating surface.

In the embodiment illustrated in FIG. 5, two such blazed diffraction gratings are used. That is, in the embodiment illustrated in FIG. 5, the diffraction grating surface of the first blazed diffraction grating Dg₃₁ is oriented to be inclined with respect to the incident light Li₁₁ incident from the light path 42, and is fixed to the light path length conversion unit 10.

In addition, the second blazed diffraction grating Dg₃₂ is disposed at a position facing the first blazed diffraction grating Dg₃₁. Specifically, the first blazed diffraction grating Dg₃₁ and the second blazed diffraction grating Dg₃₂ have the same shape. Therefore, the first blazed diffraction grating Dg₃₁ and the second blazed diffraction grating Dg₃₂ have the same groove spacing and blaze angle.

The diffraction grating surface of the first blazed diffraction grating Dg₃₁ and the diffraction grating surface of the second blazed diffraction grating Dg₃₂ are parallel to each other. Furthermore, a normal line (the normal line is, for example, Vn in FIG. 6) of the step surface of the first blazed diffraction grating Dg₃₁ and a normal line of the step surface of the second blazed diffraction grating Dg₃₂ are parallel to each other. That is, in FIG. 5, when the first blazed diffraction grating Dg₃₁ is rotated by 180° and moved in parallel, the first blazed diffraction grating Dg₃₁ overlaps with the second blazed diffraction grating Dg₃₂.

In the above configuration, the light incident from the light path 42 and reaching the first blazed diffraction grating Dg₃₁ is dispersed into lights of each wavelength by the first blazed diffraction grating Dg₃₁. In FIG. 5, routes of the lights having different wavelengths are indicated by dot-dash lines, solid lines, and broken lines. The lights traveling through the light paths indicated by the dot-dash lines have a longer wavelength than those of the lights traveling through the light paths indicated by the solid lines, and the lights traveling through the light paths indicated by the solid lines have a longer wavelength than those of the lights traveling through the light paths indicated by the broken lines. Of course, a larger number of the lights of each wavelength are continuously distributed, but only three light paths are extracted and schematically illustrated in FIG. 5.

The lights dispersed by the first blazed diffraction grating Dg₃₁ reach the second blazed diffraction grating Dg₃₂ in a dispersed state, and are diffracted by the second blazed diffraction grating Dg₃₂. The lights of each wavelength diffracted by the second blazed diffraction grating Dg₃₂ reach the mirror M₃₁ in a parallel state. Since the mirror M₃₁ is oriented perpendicularly to the traveling direction of these lights, the lights reaching the mirror M₃₁ are reflected by the mirror M₃₁ and travel in the opposite direction. Thereafter, the lights of each wavelength travel through the same light paths as the former light paths, and are brought into a non-dispersed state, at a stage of being directed from the first blazed diffraction grating Dg₃₁ toward the light path 42. Then, the non-dispersed light Li₁₂ returns to the light path 42.

Also in the above configuration, a configuration for changing the light path length for each wavelength is provided. Specifically, the second blazed diffraction grating Dg₃₂ and the mirror M₃₁ are connected to a moving mechanism (not illustrated). The moving mechanism can move the second blazed diffraction grating Dg₃₂ and the mirror M₃₁ while maintaining a state in which a relative positional relationship between the second blazed diffraction grating Dg₃₂ and the mirror M₃₁ does not change and the diffraction grating surface of the first blazed diffraction grating Dg₃₁ and the diffraction grating surface of the second blazed diffraction grating Dg₃₂ are parallel to each other. An arrow A₃₁ illustrated in FIG. 5 indicates a movable direction of the first blazed diffraction grating Dg₃₁ and the second blazed diffraction grating Dg₃₂.

When the second blazed diffraction grating Dg₃₂ and the mirror M₃₁ are moved in the movable direction, a distance between the first blazed diffraction grating Dg₃₁ and the second blazed diffraction grating Dg₃₂ varies. When the distance between the first blazed diffraction grating Dg₃₁ and the second blazed diffraction grating Dg₃₂ varies, the light path length of the light path in which the lights of each wavelength dispersed by the first blazed diffraction grating Dg₃₁ travel until the lights reach the second blazed diffraction grating Dg₃₂ varies for each wavelength. Along with this, the light path length of the light path in which the light travels from the second blazed diffraction grating Dg₃₂ to the mirror M₃₁ also varies for each wavelength. As described above, when the second blazed diffraction grating Dg₃₂ and the mirror M₃₁ are moved in a direction of the arrow A₃₁, the light path length changes. At this time, a degree of change in light path length varies for each wavelength. Therefore, according to the present embodiment, the light path length can be changed in a different change amount for each wavelength.

As in the above-described embodiment, the moving mechanism can be realized by various mechanisms for reciprocating an object in a linear direction. The configuration for changing the light path length is not limited to the configuration that reciprocates the second blazed diffraction grating Dg₃₂ and the mirror M₃₁ in the linear direction. For example, a configuration that rotates each diffraction grating while maintaining a state in which the diffraction grating surface of the first blazed diffraction grating Dg₃₁ and the diffraction grating surface of the second blazed diffraction grating Dg₃₂ are parallel to each other may be adopted.

Further, the first blazed diffraction grating Dg₃₁ may be reciprocatable in the linear direction. Of course, a mechanism that enables rotation or movement in the linear direction of the first blazed diffraction grating Dg₃₁ and the second blazed diffraction grating Dg₃₂ may be used in combination. The moving mechanism may be configured to be manually operated by the user, or may be configured to be operated in accordance with a control instruction from the measurement unit 50 or the like. In the latter case, for example, power of a motor or the like can be transmitted to the moving mechanism, and the motor or the like is operated in accordance with the control instruction.

Furthermore, shapes of the first blazed diffraction grating Dg₃₁, the second blazed diffraction grating Dg₃₂, and the mirror M₃₁ are also not limited to those in the configuration illustrated in FIG. 5. The shapes of the first blazed diffraction grating Dg₃₁ and the second blazed diffraction grating Dg₃₂ may not be completely the same, and, for example, the lengths in the direction of the groove spacing d may be different from each other.

Further, a configuration in which groove spacing di of the first blazed diffraction grating Dg₃₁ and groove spacing d₂ of the second blazed diffraction grating Dg₃₂ are different from each other can also be adopted. Specifically, in a case where the groove spacing in the first blazed diffraction grating Dg₃₁ is the groove spacing di, the diffraction order is a diffraction order n₁, the groove spacing in the second blazed diffraction grating Dg₃₂ is the groove spacing d₂, and the diffraction order is a diffraction order n2, a relationship of n₁/d₁ = n₂/d₂ may be satisfied. That is, when the relationship of n₁/d₁ = n₂/d₂ is satisfied, a diffraction angle in the first blazed diffraction grating Dg₃₁ is equal to a diffraction angle in the second blazed diffraction grating Dg₃₂. Thus, as in the example illustrated in FIG. 5, the light traveling from the light path 42 toward the first blazed diffraction grating Dg₃₁ and the lights traveling from the second blazed diffraction grating Dg₃₂ toward the mirror M₃₁ are in parallel.

Further, when the mirror M₃₁ is sufficiently larger than that illustrated in FIG. 5, the second blazed diffraction grating Dg₃₂ may be moved instead of integrally moving the second blazed diffraction grating Dg₃₂ and the mirror M₃₁.

As described above, various modifications can be adopted in the present embodiment, but, in any case, according to the present embodiment, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

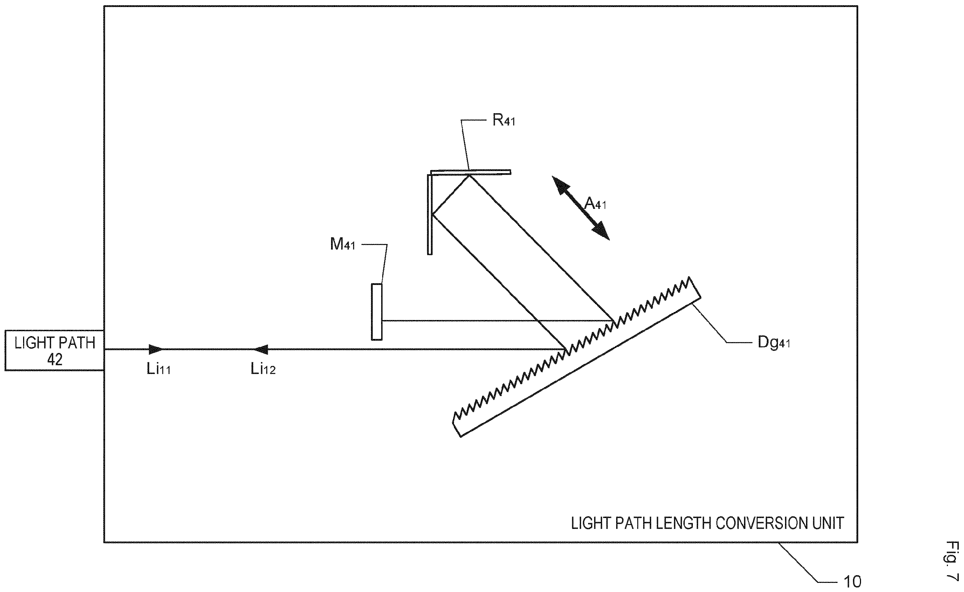

Also in the configuration illustrated in FIG. 5, the dispersed light path portion and the dispersion unit may share a member. FIG. 7 is a diagram schematically illustrating an embodiment in which the first optical member is composed of a blazed diffraction grating Dg₄₁, and the traveling direction conversion unit is composed of the blazed diffraction grating Dg₄₁, a retroreflector R₄₁ as the conversion optical member, and a mirror M₄₁ as the reflection member.

FIG. 7 is a diagram schematically illustrating a part of a configuration extracted from the light path length conversion unit 10 according to the present embodiment. The light path length conversion unit 10 includes various members in addition to members illustrated in FIG. 7. Structures similar to those in FIG. 3 are denoted by the same reference numerals as those in FIG. 3. In the configuration illustrated in FIG. 7, the light path length conversion unit 10 includes a moving mechanism (not illustrated) for moving the retroreflector R₄₁ forward and backward in a direction indicated by an arrow A₄₁ toward the blazed diffraction grating Dg₄₁.

In the configuration illustrated in FIG. 7, the light Li₁₁ incident on the light path length conversion unit 10 from the light path 42 reaches the blazed diffraction grating Dg₄₁. The light reaching the blazed diffraction grating Dg₄₁ is diffracted and dispersed by the blazed diffraction grating Dg₄₁. In FIG. 7, only light of a specific wavelength is indicated by a solid line, but the dispersed light of each wavelength travels through a light path different for each wavelength. When the light of each wavelength dispersed by the blazed diffraction grating Dg₄₁ reaches the retroreflector R₄₁, the light is reflected by the retroreflector R₄₁ and travels in a direction parallel to the incident direction and opposite to the incident direction. The light reaches the blazed diffraction grating Dg₄₁, and the light reaching the blazed diffraction grating Dg₄₁ is diffracted by the blazed diffraction grating Dg₄₁. As a result, since the light of each wavelength returns to a state of being parallel to the light before dispersion, the lights of each wavelength travel toward the mirror M₄₁ as the reflection member in a state of being parallel to each other.

When the lights of each wavelength reach the mirror M₄₁, the lights are reflected by the mirror M₄₁, become opposite, in traveling direction, to the lights before reflection, and return to the same light paths as the former light paths. That is, the lights reflected by the mirror M₄₁ are diffracted by the blazed diffraction grating Dg₄₁, reflected by the retroreflector R₄₁, and incident on the blazed diffraction grating Dg₄₁ again. When diffracted by the blazed diffraction grating Dg₄₁, the lights (dispersed lights) traveling on light paths different for each wavelength return to the state before dispersion. Then, the light Li₁₂ from the blazed diffraction grating Dg₄₁ returns to the light path 42.

As described above, in the configuration of the present embodiment, the second blazed diffraction grating Dg₃₂ and the mirror M₃₁ are excluded from the configuration illustrated in FIG. 5, and the retroreflector R₄₁ and the mirror M₄₁ are disposed instead. Since the retroreflector R₄₁ is a device that causes light to travel in the opposite direction, the configuration illustrated in FIG. 7 and the configuration illustrated in FIG. 5 are substantially almost equivalent to each other, although there is a difference that the light paths of the embodiment illustrated in FIG. 7 are folded back between the first blazed diffraction grating Dg₃₁ and the second blazed diffraction grating Dg₃₂ in FIG. 5.

Also in the configuration illustrated in FIG. 7, the dispersed lights travel through the light paths for each wavelength. Also in the present embodiment, the dispersion unit and the dispersed light path portion having such functions are realized by the blazed diffraction grating Dg₄₁, the retroreflector R₄₁, and the mirror M₄₁, and the light path length conversion unit 10 can be configured with a simple configuration.

Furthermore, also in the configuration illustrated in FIG. 7, the dispersed lights travel through different light paths, and thus the light path lengths of the dispersed lights are different for each wavelength, and the light path lengths can be changed. Specifically, the retroreflector R₄₁ is connected to a moving mechanism (not illustrated). The moving mechanism can linearly reciprocate the retroreflector R₄₁ along the direction of the arrow A₄₁, and can vary a distance between the retroreflector R₄₁ and the blazed diffraction grating Dg₄₁. When the distance between the retroreflector R₄₁ and the blazed diffraction grating Dg₄₁ varies, the light path length of the light of each wavelength changes. At this time, a degree of change in light path length varies for each wavelength. Therefore, according to the present embodiment, the light path length can be changed in a different change amount for each wavelength. Note that, also in the configuration illustrated in FIG. 7, the moving mechanism can be realized by various mechanisms for reciprocating an object in a linear direction, and another configuration, for example, a configuration that rotates the blazed diffraction grating Dg₄₁ may be used.

Also in the present embodiment, with the above configuration, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

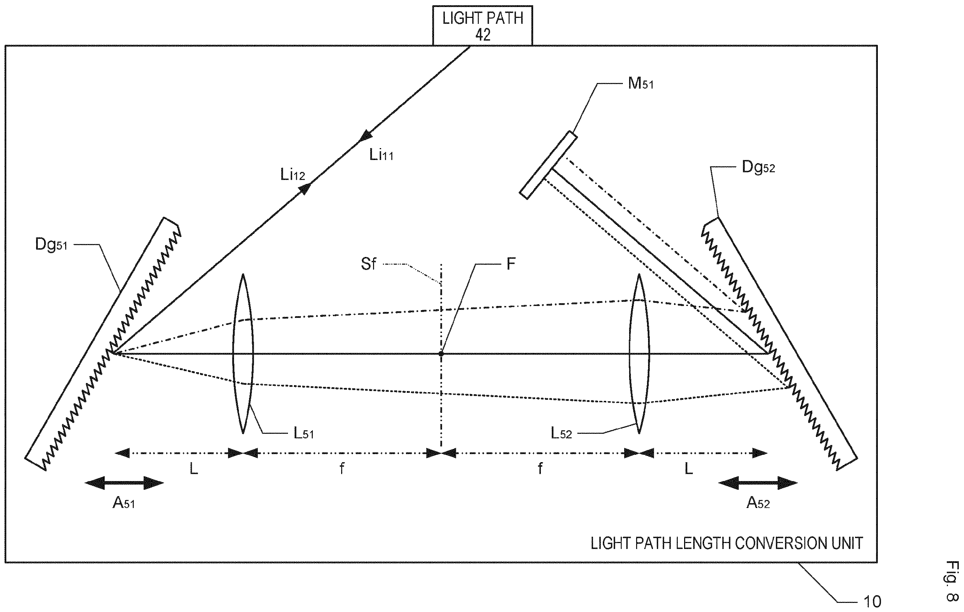

In the above-described embodiment, the second optical member may be composed of a plurality of optical members. FIG. 8 is a diagram illustrating an example in which the second optical member is composed of a member of the same type as the first optical member and a plurality of lenses. Also in a configuration illustrated in FIG. 8, the light path length conversion unit 10 includes a dispersion unit and a dispersed light path portion, the dispersion unit is composed of a first optical member, and the dispersed light path portion is composed of a traveling direction conversion unit including a second optical member that is a member of the same type as the first optical member and a reflection member that reflects light.

In the configuration illustrated in FIG. 8, the dispersion unit is a first blazed diffraction grating Dg₅₁ as the first optical member, and the dispersed light path portion includes a second blazed diffraction grating Dg₅₂ as the second optical member, a mirror M₅₁ as the reflection member, and a plurality of lenses L₅₁ and L₅₂. Note that focal lengths of the lenses L₅₁ and L₅₂ are the same.

In the embodiment illustrated in FIG. 8, a diffraction grating surface of the first blazed diffraction grating Dg₅₁ is oriented to be inclined with respect to the incident light Li₁₁ incident from the light path 42. The plurality of lenses L₅₁ and L₅₂ are disposed to face each other in parallel. That is, the plurality of lenses L₅₁ and L₅₂ are disposed such that their focal points F overlap each other and that optical axes passing through principal points of the lenses and the focal points F coincide with each other. The second blazed diffraction grating Dg₅₂ is disposed in the light path length conversion unit 10 so as to be symmetric with the first blazed diffraction grating Dg₅₁ with respect to a focal surface Sf perpendicular to the optical axis passing through the focal point F. Note that f indicated in FIG. 8 is the focal length. L is a distance between the first blazed diffraction grating Dg₅₁ and the lens L₅₁ and a distance between the second blazed diffraction grating Dg₅₂ and the lens L₅₂. That is, these two distances are equal.

In the present embodiment, the first blazed diffraction grating Dg₅₁ and the second blazed diffraction grating Dg₅₂ have the same shape. Therefore, the first blazed diffraction grating Dg₅₁ and the second blazed diffraction grating Dg₅₂ have the same groove spacing and blaze angle.

In the above configuration, the light incident from the light path 42 and reaching the first blazed diffraction grating Dg₅₁ is dispersed into lights of each wavelength by the first blazed diffraction grating Dgsi. In FIG. 8, routes of the lights having different wavelengths are indicated by dot-dash lines, solid lines, and broken lines. The lights traveling through the light paths indicated by the dot-dash lines have a longer wavelength than those of the lights traveling through the light paths indicated by the solid lines, and the lights traveling through the light paths indicated by the solid lines have a longer wavelength than those of the lights traveling through the light paths indicated by the broken lines. Of course, a larger number of the lights of each wavelength are continuously distributed, but only three light paths are extracted and schematically illustrated in FIG. 8.

The lights dispersed by the first blazed diffraction grating Dg₅₁ pass through the lenses L₅₁ and L₅₂ in a dispersed state, and travel while being refracted by the lenses L₅₁ and L₅₂. The lights output from the lens L₅₂ reach the second blazed diffraction grating Dg₅₂ and are diffracted by the second blazed diffraction grating Dg₅₂. The lights of each wavelength diffracted by the second blazed diffraction grating Dg₅₂ reach the mirror M₅₁ in a parallel state. Since the mirror M₅₁ is oriented perpendicularly to the traveling direction of these lights, the lights reaching the mirror M₅₁ are reflected by the mirror M₅₁ and travel in the opposite direction. Thereafter, the lights of each wavelength travel through the same light paths as the former light paths, and are brought into a non-dispersed state, at a stage of being directed from the first blazed diffraction grating Dg₅₁ toward the light path 42. Then, the non-dispersed light Li₁₂ returns to the light path 42.

Also in the above configuration, a configuration for changing the light path length for each wavelength is provided. Specifically, the first blazed diffraction grating Dg₅₁ and the second blazed diffraction grating Dg₅₂ are connected to a moving mechanism (not illustrated). The moving mechanism can move the first blazed diffraction grating Dg₅₁ and the second blazed diffraction grating Dg₅₂ in directions of arrows A₅₁ and A₅₂ while maintaining a state in which the first blazed diffraction grating Dg₅₁ and the second blazed diffraction grating Dg₅₂ are symmetric with respect to the focal surface Sf. In addition, the moving mechanism performs the movement while maintaining a state in which the distance L between the first blazed diffraction grating Dg₅₁ and the lens L₅₁ is equal to the distance L between the second blazed diffraction grating Dg₅₂ and the lens L₅₂. When the distance L varies, the mirror M₅₁ may move integrally with the second blazed diffraction grating Dg₅₂, or the mirror M₅₁ may not move. That is, if the mirror M₅₁ is sufficiently large, the mirror M₅₁ may be fixed.

When a magnitude of the distance L is varied, the light path length of the light path through which the light of each wavelength dispersed by the first blazed diffraction grating Dg₅₁ travels until reaching the second blazed diffraction grating Dg₅₂ varies for each wavelength. Along with this, the light path length of the light path in which the light travels from the second blazed diffraction grating Dg₅₂ to the mirror M₅₁ also varies for each wavelength. As described above, when the magnitude of the distance L is varied, the light path length changes. At this time, a degree of change in light path length varies for each wavelength. Therefore, according to the present embodiment, the light path length can be changed in a different change amount for each wavelength.

As in the above-described embodiment, the moving mechanism can be realized by various mechanisms for reciprocating an object in a linear direction. The configuration for changing the light path length is not limited to the configuration that reciprocates the first blazed diffraction grating Dg₅₁ and the second blazed diffraction grating Dg₅₂ in the linear direction. For example, a configuration that rotates each diffraction grating while maintaining a state in which the first blazed diffraction grating Dg₅₁ and the second blazed diffraction grating Dg₅₂ are symmetric with respect to the focal surface Sf.

Of course, a mechanism that enables rotation or movement in the linear direction of the first blazed diffraction grating Dg₅₁ and the second blazed diffraction grating Dg₅₂ may be used in combination. The moving mechanism may be configured to be manually operated by the user, or may be configured to be operated in accordance with a control instruction from the measurement unit 50 or the like. In the latter case, for example, power of a motor or the like can be transmitted to the moving mechanism, and the motor or the like is operated in accordance with the control instruction.

Furthermore, shapes of the first blazed diffraction grating Dg₅₁ and the second blazed diffraction grating Dg₅₂ are also not limited to those in the configuration illustrated in FIG. 8. The shapes of the first blazed diffraction grating Dg₅₁ and the second blazed diffraction grating Dg₅₂ may not be completely the same, and, for example, the lengths in the direction of the groove spacing d may be different from each other. Also, in a case where the groove spacing in the first blazed diffraction grating Dg₅₁ is the groove spacing di, the diffraction order is a diffraction order n₁, the groove spacing in the second blazed diffraction grating Dg₅₂ is the groove spacing d₂, and the diffraction order is a diffraction order n2, a relationship of n₁/d₁ = n₂/d₂ may be satisfied.

As described above, various modifications can be adopted in the present embodiment, but, in any case, according to the present embodiment, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

Also in the configuration illustrated in FIG. 8, the dispersed light path portion and the dispersion unit may share a member. FIG. 8 is a diagram schematically illustrating an embodiment in which the first optical member is composed of a blazed diffraction grating Dg₆₁, and the traveling direction conversion unit is composed of the blazed diffraction grating Dg₆₁, a lens L₆₁ as the conversion optical member, a mirror M₆₂ as the reflection member, and a mirror M₆₁ as a reflection member that returns light from the blazed diffraction grating Dg₆₁ to the blazed diffraction grating Dg₆₁ again.

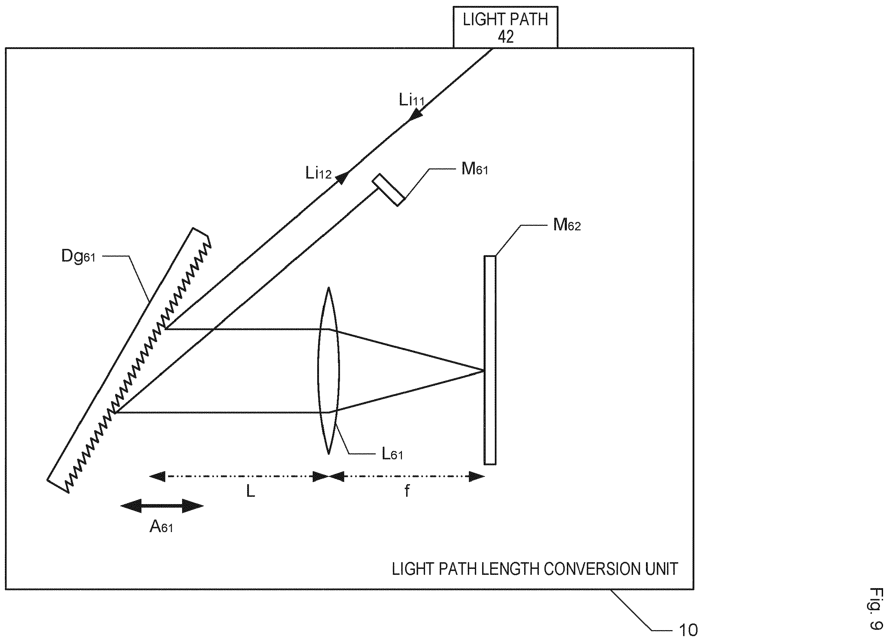

FIG. 9 is a diagram schematically illustrating a part of a configuration extracted from the light path length conversion unit 10 according to the present embodiment. The light path length conversion unit 10 includes various members in addition to members illustrated in FIG. 9. Structures similar to those in FIG. 3 are denoted by the same reference numerals as those in FIG. 3. In the configuration illustrated in FIG. 9, the light path length conversion unit 10 includes a moving mechanism (not illustrated) for moving the blazed diffraction grating Dg₆₁ forward and backward in a direction indicated by an arrow A₆₁ toward the lens L₆₁.

In the configuration illustrated in FIG. 9, the light Li₁₁ incident on the light path length conversion unit 10 from the light path 42 reaches the blazed diffraction grating Dg₆₁. The light reaching the blazed diffraction grating Dg₆₁ is diffracted and dispersed by the blazed diffraction grating Dg₆₁. In FIG. 9, only light of a specific wavelength is indicated by a solid line, but the dispersed light of each wavelength travels through a light path different for each wavelength. When the light of each wavelength dispersed by the blazed diffraction grating Dg₆₁ reaches the lens L₆₁, the light is refracted by the lens L₆₁ and travels toward the mirror M₆₂.

Further, the light is reflected by the mirror M₆₂ and travels toward the lens L₆₁ again. When the light reaches the lens L₆₁, the light is refracted by the lens L₆₁ and travels toward the blazed diffraction grating Dg₆₁. The light reaching the blazed diffraction grating Dg₆₁ is diffracted by the blazed diffraction grating Dg₆₁. As a result, since the light of each wavelength returns to a state of being parallel to the light before dispersion, the lights of each wavelength travel toward the mirror M₆₁ as the reflection member in a state of being parallel to each other.

When the lights of each wavelength reach the mirror M₆₁, the lights are reflected by the mirror M₆₁, become opposite, in traveling direction, to the lights before reflection, and return to the same light paths as the former light paths. That is, the lights reflected by the mirror M₆₁ are diffracted by the blazed diffraction grating Dg₆₁, pass through the lens L₆₁, are reflected again by the mirror M₆₂, and incident on the blazed diffraction grating Dg₆₁ again via the lens L₆₁. When diffracted by the blazed diffraction grating Dg₆₁, the lights (dispersed lights) traveling on light paths different for each wavelength return to the state before dispersion. Then, the non-dispersed light Li₁₂ returns to the light path 42.

As described above, in the configuration of the present embodiment, the lens L₅₂, the second blazed diffraction grating Dg₅₂, and the mirror M₅₁ are excluded from the configuration illustrated in FIG. 8, and instead, the mirror M₆₂ and the mirror M₆₁ are disposed. Note that a reflecting surface of the mirror M₆₂ exists at the same position as the focal surface Sf in the configuration illustrated in FIG. 8. Since the mirror M₆₂ is a device that causes light to travel in the opposite direction, the configuration illustrated in FIG. 9 and the configuration illustrated in FIG. 8 are substantially almost equivalent to each other, although there is a difference that the light paths of the embodiment illustrated in FIG. 9 are folded back between the first blazed diffraction grating Dg₅₁ and the second blazed diffraction grating Dg₅₂ in FIG. 8.

Also in the configuration illustrated in FIG. 9, the dispersed lights travel through the light paths for each wavelength. Also in the present embodiment, the dispersion unit and the dispersed light path portion having such functions are realized by the blazed diffraction grating Dg₆₁, the lens L₆₁, and the mirrors M₆₁ and M₆₂, and the light path length conversion unit 10 can be configured with a simple configuration.

Furthermore, also in the configuration illustrated in FIG. 9, the dispersed lights travel through different light paths, and thus the light path lengths of the dispersed lights are different for each wavelength, and the light path lengths can be changed. Specifically, the blazed diffraction grating Dg₆₁ is connected to a moving mechanism (not illustrated). The moving mechanism can linearly reciprocate the blazed diffraction grating Dg₆₁ along the direction of the arrow A₆₁, and can vary a distance between the blazed diffraction grating Dg₆₁ and the lens L₆₁. When the distance between the blazed diffraction grating Dg₆₁ and the lens L₆₁ varies, the light path length of the light of each wavelength changes. At this time, a degree of change in light path length varies for each wavelength. Therefore, according to the present embodiment, the light path length can be changed in a different change amount for each wavelength. Note that, also in the configuration illustrated in FIG. 9, the moving mechanism can be realized by various mechanisms for reciprocating an object in a linear direction, and another configuration, for example, a configuration that rotates the blazed diffraction grating Dg₆₁ may be used.

Also in the present embodiment, with the above configuration, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

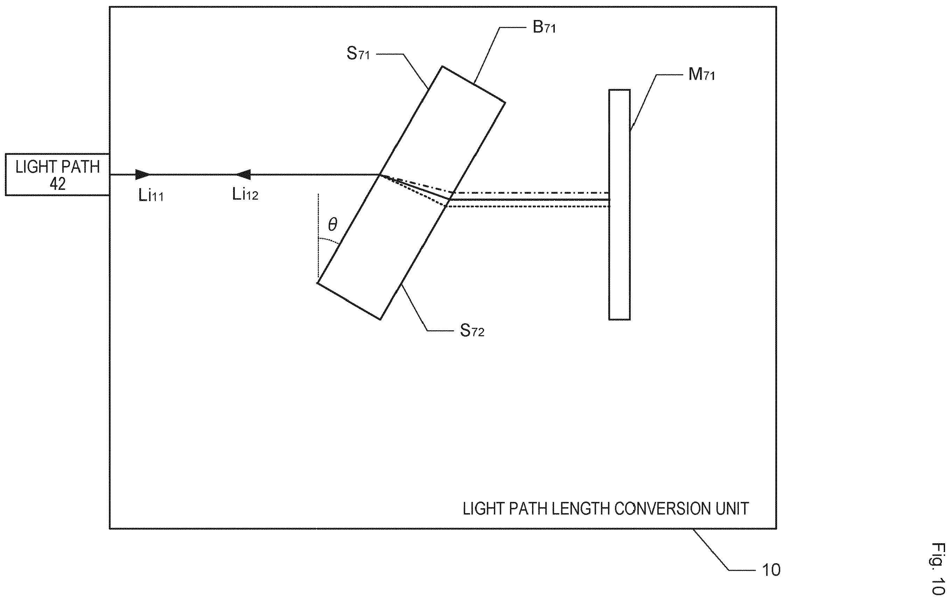

Furthermore, the dispersion unit may be a parallel flat plate. That is, various optical members that disperse light using refraction at an interface between air and the dispersion unit may be used. FIG. 10 illustrates an embodiment in which the dispersion unit is composed of a parallel flat plate B₇₁ and the dispersed light path portion is composed of a mirror M₇₁.

Also in the configuration illustrated in FIG. 10, some of members and the like constituting the light path length conversion unit 10, such as a moving mechanism, are omitted. In the embodiment illustrated in FIG. 10, a plane S₇₁ of the parallel flat plate B₇₁ is oriented to be inclined with respect to the incident light Li₁₁ incident from the light path 42, and is fixed to the light path length conversion unit 10.

The light incident from the light path 42 and reaching the parallel flat plate B₇₁ is refracted at the plane S₇₁ which is an interface between air and the parallel flat plate B₇₁, and is dispersed into lights of each wavelength. In FIG. 10, routes of the lights having different wavelengths are indicated by dot-dash lines, solid lines, and broken lines. The lights traveling through the light paths indicated by the dot-dash lines have a longer wavelength than those of the lights traveling through the light paths indicated by the solid lines, and the lights traveling through the light paths indicated by the solid lines have a longer wavelength than those of the lights traveling through the light paths indicated by the broken lines. Of course, a larger number of the lights of each wavelength are continuously distributed, but only three light paths are extracted and schematically illustrated in FIG. 10.

The lights dispersed by the parallel flat plate B₇₁ travel in the parallel flat plate B₇₁ in a dispersed state, and are refracted again at the interface between the parallel flat plate B₇₁ and air. A plane S₇₂ which is the interface is parallel to the plane S₇₁. Therefore, the lights of each wavelength refracted by the plane S₇₂ reach the mirror M₇₁ in a parallel state. Since the mirror M₇₁ is oriented perpendicularly to the traveling direction of these lights, the lights reaching the mirror M₇₁ are reflected by the mirror M₇₁ and travel in the opposite direction. Thereafter, the lights of each wavelength travel through the same light paths as the former light paths, and are brought into a non-dispersed state, at a stage of being directed from the parallel flat plate B₇₁ toward the light path 42. Then, the non-dispersed light Li₁₂ returns to the light path 42.

Also in the above configuration, a configuration for changing the light path length for each wavelength is provided. Specifically, the parallel flat plate B₇₁ is connected to a moving mechanism (not illustrated), and can vary an inclination angle θ of the plane S₇₁ with respect to the incident light Li₁₁.

When a magnitude of the inclination angle θ is varied, the light path length of the light path through which the light of each wavelength dispersed by the plane S₇₁ of the parallel flat plate B₇₁ travels until reaching the mirror M₇₁ varies for each wavelength. As described above, when the magnitude of the inclination angle θ is varied, the light path length changes. At this time, a degree of change in light path length varies for each wavelength. Therefore, according to the present embodiment, the light path length can be changed in a different change amount for each wavelength.

As in the above-described embodiment, the moving mechanism can be realized by various mechanisms for rotating an object. The moving mechanism may be configured to be manually operated by the user, or may be configured to be operated in accordance with a control instruction from the measurement unit 50 or the like. In the latter case, for example, power of a motor or the like can be transmitted to the moving mechanism, and the motor or the like is operated in accordance with the control instruction.

As described above, various modifications can be adopted in the present embodiment, but, in any case, according to the present embodiment, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

In the above-described embodiment, the light path length conversion unit is provided in the reference arm on the light path 42 side through which the reference lights divided by the division unit 30 travel, but may be provided in the measurement arm on the light path 43 side through which the measurement lights divided by the division unit 30 travel. FIG. 11 is a diagram schematically illustrating a configuration in which a light path length conversion unit 100 is provided in the light path 43 between the division unit 30 in the measurement arm and the measurement target.

In the embodiment illustrated in FIG. 11, the same components as those in FIG. 1 are denoted by the same reference numerals as those in FIG. 1. A mirror 11 that reflects the reference light is provided in a portion where the light path length conversion unit 10 exists in the configuration illustrated in FIG. 1. The light path length conversion unit 100 illustrated in FIG. 11 can vary the light path length of the light before being emitted to the measurement target for each wavelength in the light path 43 that is the measurement arm. As a configuration for this, various configurations can be adopted, and, also here, the light path length conversion unit 100 is configured to include a dispersion unit that disperses light for each wavelength and a dispersed light path portion that forms a light path having a light path length different for each wavelength.

However, in the measurement arm, the measurement light is reflected by the measurement target, and thus the light path length conversion unit 100 does not include a function of reflecting light and returning the light in the opposite direction. Specifically, the dispersion unit has a first optical member that disperses light into lights of each wavelength. Furthermore, the dispersed light path portion has a traveling direction conversion unit that receives lights of each wavelength dispersed by the first optical member, converts traveling directions of the lights of each wavelength, and returns the lights to light that is not dispersed for each wavelength. That is, in the dispersed light path portion, the routes of the lights of each wavelength are converted by using the traveling direction conversion unit, but the lights are caused to travel without returning to the opposite direction by reflection or the like.

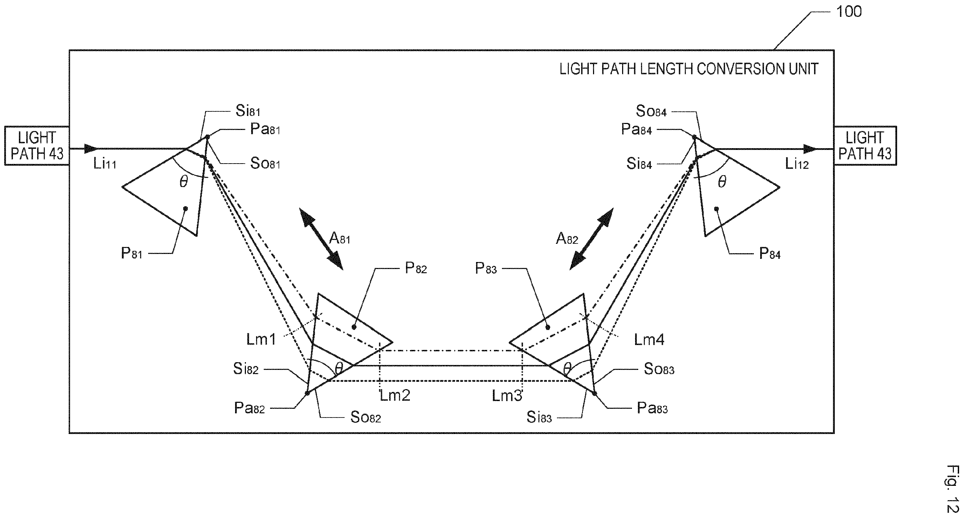

FIG. 12 is a diagram schematically illustrating a part of a configuration according to an example of the light path length conversion unit 100. In the configuration illustrated in FIG. 12, the first optical member is composed of a prism Psi. The traveling direction conversion unit is composed of a prism P₈₂, a prism P₈₃, and a prism P₈₄. The light path length conversion unit 100 includes various members in addition to these members. For example, the light Li₁₁ directed from the light path 43 toward the prism P₈₁ is parallel light, and the light Li₁₁ becomes parallel light by disposing a collimator (not illustrated) or the like between the light path 43 and the prism Psi. In addition, the light path length conversion unit 100 includes a moving mechanism (not illustrated) and the like.

In the configuration illustrated in FIG. 12, when the light Li₁₁ incident on the light path length conversion unit 100 from the light path 43 and directed to the prism Psi reaches an incident surface Si₈₁ of the prism P₈₁, the light Li₁₁ is dispersed by refraction, and the dispersed light travels in a traveling direction different for each wavelength. Further, when the light of each wavelength reaches an outgoing surface So₈₁ of the light from the prism Psi, the light is refracted again and travels in the air. In FIG. 12, routes of the lights having different wavelengths are indicated by dot-dash lines, solid lines, and broken lines. The lights traveling through the light paths indicated by the dot-dash lines have a longer wavelength than those of the lights traveling through the light paths indicated by the solid lines, and the lights traveling through the light paths indicated by the solid lines have a longer wavelength than those of the lights traveling through the light paths indicated by the broken lines. Of course, a larger number of the lights of each wavelength are continuously distributed, but only three light paths are extracted and schematically illustrated in FIG. 12.

When the light output from the prism Psi reaches the prism P₈₂, the light is refracted by an incident surface Si₈₂ of the light to the prism P₈₂, travels inside the prism P₈₂, is refracted again by the outgoing surface So₈₂ of the light from the prism P₈₂, and reaches the prism P₈₃ in a state where the lights of each wavelength are parallel to each other. When the light of each wavelength reaches the prism P₈₃, the light is refracted by an incident surface Si₈₃ of the light to the prism P₈₃, travels inside the prism P₈₃, is refracted again by an outgoing surface So₈₃ of the light from the prism P₈₃, and travels toward the prism P₈₄.

When the light of each wavelength reaches the prism P₈₄, the light is refracted by an incident surface Si₈₄ of the light to the prism P₈₄, travels inside the prism P₈₄, is refracted again by an outgoing surface So₈₄ of the light from the prism P₈₄, and is output to the light path 43 as non-dispersed light. In the present embodiment, the prisms Psi, P₈₂, P₈₃, and P₈₄ are the same medium and have the same shape. In the example illustrated in FIG. 12, the prisms Psi, P₈₂, P₈₃, and P₈₄ are triangular prisms, and axes of the triangular prisms are parallel to each other. In addition, a plane perpendicular to the axes of the triangular prisms (a plane parallel to the paper surface of FIG. 12) is parallel to optical axes of the lights Li₁₁ and Li₁₂.

Further, the incident surface Si₈₁ of the light to the prism P₈₁ and the outgoing surface So₈₂ of the light from the prism P₈₂ are oriented in parallel. In addition, the outgoing surface So₈₁ of the light from the prism P₈₁ and the incident surface Si₈₂ of the light to the prism P₈₂ are oriented in parallel. Further, the incident surface Si₈₃ of the light to the prism P₈₃ and the outgoing surface So₈₄ of the light from the prism P₈₄ are oriented in parallel. In addition, the outgoing surface So₈₃ of the light from the prism P₈₃ and the incident surface Si₈₃ of the light to the prism P₈₃ are oriented in parallel. In addition, the outgoing surface So₈₂ of light from the prism P₈₂ and the incident surface Si₈₃ of light to the prism P₈₃ are symmetric with respect to a plane equidistant from axes of the prisms P₈₂ and P₈₃, which are triangular prisms.

Therefore, the light incident on the prism Psi from the light path 43 and the light directed from the prism P₈₂ to the prism P₈₃ are parallel. Further, the light incident on the prism P₈₃ and the light from the prism P₈₄ toward the light path 43 are parallel to each other. In addition, lights dispersed by the light passing through the prism Psi return to a non-dispersed state after passing through the prisms P₈₂, P₈₃, and P₈₄. Therefore, the lights are maintained in a dispersed state in a process in which the lights pass through the prisms Psi to P₈₄.

As described above, in the light path length conversion unit 100, the light input from the light path 43 to the prism Psi is dispersed into the lights of each wavelength. In addition, the dispersed lights of each wavelength travel through different light paths. In the present embodiment, the dispersion unit and the dispersed light path portion having such functions are realized by the prisms Psi to P₈₄, and the light path length conversion unit 100 can be configured with a simple configuration.

In the present embodiment, the dispersed lights travel through different light paths, and thus the light path lengths of the dispersed lights are different for each wavelength. In the configuration illustrated in FIG. 12, a light path length between two-dot chain lines Lm1 and Lm2 and a light path length between two-dot chain lines Lm3 and Lm4 are different for each wavelength. Furthermore, in the present embodiment, the light path length for each wavelength can be changed. The configuration for changing the light path length may be realized by various configurations, and, for example, it is possible to change the light path length for each wavelength of dispersed light by changing at least one of a distance between or directions of the dispersion unit and the traveling direction conversion unit.

In the present embodiment, the prisms P₈₂ and P₈₃ constituting the traveling direction conversion unit can be moved in synchronization. Specifically, the prism P₈₂ and the prism P₈₃ are connected to a moving mechanism (not illustrated). The moving mechanism can move the prism P₈₂ in a direction of an arrow A₈₁ while maintaining a state in which the outgoing surface So₈₁ of the prism P₈₁ is parallel to the incident surface Si₈₂ of the prism P₈₂. Further, the moving mechanism can move the prism P₈₃ in a direction of an arrow A₈₂ while maintaining a state in which the outgoing surface So₈₃ of the prism P₈₃ and the incident surface Si₈₄ of the prism P₈₄ are parallel to each other.

Further, when the prism P₈₂ moves in a direction of approaching the prism P₈₁, the prism P₈₃ moves in a direction of approaching the prism P₈₄. When the prism P₈₂ moves in a direction away from the prism Psi, the prism P₈₃ moves in a direction away from the prism P₈₄. An amount of movement at this time is the same between the prisms P₈₂ and P₈₃. In the above configuration, when the prism P₈₂ is moved in the direction of the arrow Asi and the prism P₈₃ is moved in the direction of the arrow A₈₂, the light path length changes. At this time, a degree of change in light path length varies for each wavelength. Therefore, according to the present embodiment, the light path length can be changed in a different change amount for each wavelength.

Also in the present embodiment, the moving mechanism can be realized by various mechanisms for reciprocating an object in a linear direction. Further, the configuration for changing the light path length is not limited to the configuration that reciprocates the prisms P₈₂ and P₈₃ in the linear direction. For example, vertexes Pa₈₁ and Pa₈₂ of the prisms P₈₁ and P₈₂ may be set as rotation axes, and orientations of the prisms Psi and P₈₂ may be changed so as to rotate by the same angle in the same rotation direction. In this case, vertexes Pa₈₃ and Pa₈₄ of the prisms P₈₃ and P₈₄ may be set as rotation axes, and orientations of the prisms P₈₃ and P₈₄ may be changed so as to rotate by the same angle in a reverse rotation direction as the prisms Psi and P₈₂. That is, the prisms Psi to P₈₄ may be rotated while maintaining a state in which the outgoing surface So₈₁ of the prism Psi is parallel to the incident surface Si₈₂ of the prism P₈₂, and maintaining the state in which the outgoing surface So₈₃ of the prism P₈₃ is parallel to the incident surface Si₈₄ of the prism P₈₄.

Further, the prisms Psi and P₈₄ may be reciprocatable in the linear direction. Needless to say, a mechanism that enables the prisms to rotate or move in the linear direction may be used in combination. Note that the moving mechanism may be configured to be manually operated by the user, or may be configured to be operated in accordance with a control instruction from the measurement unit 50 or the like. In the latter case, for example, power of a motor or the like can be transmitted to the moving mechanism, and the motor or the like is operated in accordance with the control instruction.

Furthermore, shapes of the prisms Psi to P₈₄ are not limited to those in the configuration illustrated in FIG. 12. For example, the shapes of the prisms P₈₁ to P₈₄ may not be completely the same. That is, in the prisms Psi to P₈₄, it is sufficient that the angles θ at the vertexes Pa₈₁ to Pa₈₄ be the same, that the outgoing surface So₈₁ of the prism P₈₁ and the incident surface Si₈₂ of the prism P₈₂ be parallel, that the outgoing surface So₈₃ of the prism P₈₃ and the incident surface Si₈₄ of the prism P₈₄ be parallel, and that the outgoing surface So₈₂ of the light from the prism P₈₂ and the incident surface Si₈₃ of the light to the prism P₈₃ be symmetric with respect to a plane equidistant from axes of the prisms P₈₂ and P₈₃ which are triangular prisms. Therefore, for example, triangles formed by the prisms Psi to P₈₄ in FIG. 12 may not be congruent.

As described above, various modifications can be adopted in the present embodiment, but, in any case, according to the present embodiment, the light path length for each wavelength can be changed by the moving mechanism. Therefore, the OCT device 1 according to the present embodiment can adjust dispersion characteristics and easily perform adjustment for sufficiently broadening the distribution of the interference signal. Furthermore, even if the wavelength range of the light used in the OCT device 1 slightly differs for each individual due to individual differences of the light source 20, setting differences, and the like, adjustment can be performed so as not to cause a difference in characteristics of the interference signal for each individual of the OCT device 1 by performing adjustment after manufacturing the OCT device 1.

Note that the configuration illustrated in FIG. 12 is similar to the configuration illustrated in FIG. 3. That is, if, in the configuration illustrated in FIG. 3, the mirror M₁₁ disposed at an intermediate point of the light path after dispersion is removed, and the same optical components as the optical components (prisms P₁₁ and P₁₂) disposed between the light path 42 and the mirror M₁₁ are disposed at positions symmetrical with respect to the reflection surface of the mirror M₁₁, the configuration is equivalent to that in FIG. 12. When the same operations are performed on the configuration in FIG. 4, 5, 7, 8, 9, or 10, the light path length conversion unit 100 disposed in the measurement arm can be configured.

In each of the above-described embodiments, the light source only needs to be able to output light including a plurality of wavelengths. That is, the light before dispersion should include a plurality of wavelengths so that a light path having a light path length different for each wavelength can be formed. The wording "a plurality of wavelengths" corresponds to a state in which the number of wavelengths included in the light is not one, and may be light in which the wavelengths are continuously distributed in a certain wavelength band, or may be light in which the wavelengths are discretely distributed. In addition, a width of the wavelength band is not limited. The full width at half maximum may be various, such as 30 nm and 120 nm, and the center wavelength is also not limited to 840 nm as described above. For example, light sources having various wavelength bandwidths in which the center wavelength is 1064 nm, 1310 nm, 1550 nm, or 1700 nm may be used. Note that, as described above, the optical components included in the OCT device 1 may be different from those of the above-described embodiments, and the OCT device 1 may be of SS-OCT. In this case, the light source may be a light source whose output wavelength changes with time, that is, a wavelength swept light source.

The division unit only needs to be able to divide the light output from the light source into the reference light and the measurement light. That is, since the OCT device is a device that detects the interference light between the reflected light and the reference light, the division unit only needs to be able to divide the light output from a single light source into the reference light and the measurement light and cause the lights to travel through different light paths. Therefore, the configuration of the division unit and the optical system for detecting the interference light is not limited to the Michelson's interferometer as described above. For example, a balanced Michelson's interferometer, a Mach-Zehnder interferometer, or the like may be used.

The measurement arm should form a light path of the measurement light with which the measurement target is irradiated and the reflected light from the measurement target, which is generated by the measurement light. That is, an arbitrary optical system that irradiates the measurement target with the measurement light and causes the reflected light from the measurement light to travel so that the reflected light interferes with the reference light can serve as the measurement arm. Of course, the optical component constituting the optical system is not limited, and various optical components such as arbitrary lenses, optical fibers, mirrors, galvanometers, optical couplers, optical circulators, polarization controllers, and beam splitters may be used.

The reference arm should form a light path of the reference light. That is, the reference light is light that interferes with the reflected light from the measurement target, and the reference arm only needs to be able to cause the light to travel so that the measurement target can be measured by interference with the reference light. Therefore, as in the above-described embodiments, the reference light may be reflected by the mirror, or the light path may be formed so that the reference light having the same phase as that of the configuration can be obtained.

The measurement unit only needs to be able to measure the measurement target on the basis of the interference light between the reflected light and the reference light. That is, it is sufficient that the measurement unit can perform various measurements on the measurement target on the basis of analysis of the interference light or the like. Therefore, in addition to the configuration in which a tomographic image is generated and displayed on a display unit as described above, the measurement target may be measured in various modes, and the measurement unit may be used in various modes. In addition, the measurement target is not limited to the eye to be examined, and may be a part of the skin or the like, or a tissue or the like of a body of an animal other than a human. Various measurement targets may be measured.

The dispersion unit only needs to be able to disperse light into lights of each wavelength. Therefore, the dispersion unit can be realized by various optical members that cause the lights to travel in different directions for each wavelength of light. The first optical member as such an optical member may be realized by various members other than the diffraction grating and the prism, and may be, for example, a virtual imaged phased array (VIPA), a grism, or the like, or a combination thereof. In addition, the second optical member is not limited to the diffraction grating or the prism, and may be a virtual imaged phased array (VIPA), a grism, or the like, or a combination thereof. In addition, various modes can be adopted as modes of these optical members. For example, the diffraction grating is not limited to a blazed diffraction grating, and may be various modes, or may be either a reflection type or a transmission type. Furthermore, the configuration for adjusting the light path length is not limited to the above-described embodiments, and may include, for example, a larger number of optical components. Specifically, when the prisms P₁₁ and P₁₂ between the mirror M₁₁ and the light path 42 illustrated in FIG. 3 are considered as a set of optical components, a plurality of sets of optical components may exist between the mirror M₁₁ and the light path 42.

The dispersed light path portion should form light paths of dispersed lights, the light paths having a light path length different for each wavelength. That is, the lights dispersed for each wavelength should pass through different light paths, and the light path lengths of the lights of each wavelength traveling through the light paths should be different from each other. According to this configuration, the interference light can be analyzed in a state where the light path length is different for each wavelength of light. Therefore, the intensity of the interference signal with respect to the spatial frequency can be configured to be broad as compared with a case where the light path length for each wavelength of light is the same. Further, the dispersed light path portion may be provided in at least one of the measurement arm and the reference arm, or may be provided in both of the measurement arm and the reference arm.

In the light path formed by the dispersed light path portion, the light path length for each wavelength of light may be variable or fixed. When it is possible to change the light path length as in the above-described embodiments, it is possible to change the light path length and adjust it for various purposes including fine adjustment in an operation process, for example, after shipment of the OCT device. Of course, the light path length may be adjusted and fixed before shipment.

On the other hand, even if the light path length is configured not to be variable but to be fixed in the OCT device, the light path length for each wavelength can be easily adjusted by changing at least one of the distance between or the orientations of the dispersion unit and the traveling direction conversion unit at the design stage. In addition, the light path length for each wavelength can be easily adjusted by adjusting the attachment position, angle, and the like of the optical component before shipment in the OCT device. Therefore, for example, it is possible to easily adjust the light path length for each wavelength as compared with a configuration in which the light path length for each wavelength is adjusted by the thickness of the dispersion member.

The light path length for each wavelength may be determined by various methods. For example, in a configuration using DEFR, when the intensity of the interference signal before wavelength dispersion correction is plotted on a graph with the spatial frequency on the horizontal axis, the distribution must be widened. In an OCT device having a narrow bandwidth of a light source, the distribution is narrower than that in an OCT device having a wide bandwidth. Therefore, in the OCT device having a narrow bandwidth, the difference in light path length of the light of each wavelength may be made larger than that in the OCT device having a wide bandwidth, so that the distribution may be widened.

Furthermore, the optical system constituting the OCT may be provided for each measurement target. For example, as disclosed in JP 2018-47099 A, two optical systems for the fundus and the anterior ocular segment, respectively, as measurement targets may be provided. In this case, the dispersion unit and the dispersed light path portion may be provided in each optical system, or may be provided in one of the optical systems, or may be provided in various modes.

## Claims

1. An OCT device comprising:
a light source configured to output light including a plurality of wavelengths;
a division unit configured to divide the light output from the light source into reference light and measurement light;
a measurement arm configured to form a light path of the measurement light with which a measurement target is irradiated and reflected light from the measurement target, which is generated by the measurement light;
a reference arm confirmed to form a light path of the reference light;
a measurement unit configured to Fourier-transform interference light between the reflected light and the reference light to measure the measurement target; and
at least one of the measurement arm and the reference arm includes a dispersion unit configured to disperse light into lights of each wavelength and a dispersed light path portion configured to form light paths of dispersed lights, the light paths having a light path length different for each wavelength.

2. The OCT device according to claim 1, wherein
the measurement unit is configured to increase a measurement range by performing, on the interference light, correction of wavelength dispersion and removal of a complex conjugate signal of an interference signal.

3. The OCT device according to claim 1 or 2, wherein
the dispersion unit has
a first optical member configured to disperse light into lights of each wavelength, and
the dispersed light path portion has
a traveling direction conversion unit configured to receive the lights of each wavelength dispersed by the first optical member, convert traveling directions of the lights of each wavelength to opposite directions while maintaining a dispersed state, and return the lights to the first optical member.

4. The OCT device according to claim 3, wherein
a light path length for each wavelength of the dispersed light is changed by changing at least one of a distance between, or orientations of, the dispersion unit and the traveling direction conversion unit.

5. The OCT device according to claim 3 or 4, wherein
the traveling direction conversion unit includes:
a second optical member that includes a member of a same type as the first optical member, is configured to convert traveling directions of the lights of each wavelength output from the first optical member into traveling directions parallel to each other, and output the lights; and
a reflection member configured to reflect the lights of each wavelength output from the second optical member, the lights being parallel to each other, and return the lights to the second optical member.

6. The OCT device according to claim 5, wherein
the first optical member and the second optical member are
any or a combination of a diffraction grating, a prism, a virtual imaged phased array (VIPA), and a grism.

7. The OCT device according to claim 5, wherein
the first optical member is
any or a combination of a diffraction grating, a prism, a virtual imaged phased array (VIPA), and a grism, and
the second optical member is
a member of a same type as the first optical member and a plurality of lenses.

8. The OCT device according to claim 3 or 4, wherein
the traveling direction conversion unit includes:
a conversion optical member configured to convert the lights of each wavelength output from the first optical member into lights having opposite traveling directions and traveling on different light paths, and return the lights to the first optical member; and
a reflection member configured to reflect the lights of each wavelength parallel in traveling direction to each other, and return the lights to the first optical member, the lights of each wavelength parallel in traveling direction to each other being included in the lights output from the first optical member by the returned light.

9. The OCT device according to claim 8, wherein
the first optical member is
any or a combination of a diffraction grating, a prism, a virtual imaged phased array (VIPA), and a grism, and
the conversion optical member is
a retroreflector.

10. The OCT device according to claim 8, wherein
the first optical member is
any or a combination of a diffraction grating, a prism, a virtual imaged phased array (VIPA), and a grism, and
the conversion optical member is
a lens and a reflection member.

11. The OCT device according to claim 1, wherein
the dispersion unit is
a first blazed diffraction grating,
the dispersed light path portion includes:
a second blazed diffraction grating that has a diffraction grating surface facing the first blazed diffraction grating, is parallel to the first blazed diffraction grating, has a same groove spacing and blaze angle, and has a normal line of a step surface which is parallel to a normal line of a step surface of the first blazed diffraction grating; and
a reflection member oriented in a direction perpendicular to traveling directions of lights of each wavelength traveling in directions parallel to each other from the second blazed diffraction grating, and
a light path length for each wavelength of light dispersed by the first blazed diffraction grating can be changed by moving at least one of the diffraction grating surface of the first blazed diffraction grating and the diffraction grating surface of the second blazed diffraction grating while maintaining a parallel state.

12. The OCT device according to claim 1 or 2, wherein
the dispersion unit has
a first optical member configured to disperse light into the lights of each wavelength, and
the dispersed light path portion has
a traveling direction conversion unit configured to receive the lights of each wavelength dispersed by the first optical member, convert traveling directions of the lights of each wavelength, and return the lights to light that is not dispersed for each wavelength.
